# EUROPEAN PATENT SPECIFICATION

(11) **EP 4 168 123 B1**
(45) Date of publication and mention of the grant of the patent: **27.08.2025**
(21) Application number: 21826551.0
(22) Date of filing: 17.06.2021
(51) Int. Cl.: A61K 31/69, A61P 35/00, C12Q 1/6886, G01N 33/574, G16H 50/20, A61K 38/05, A61K 38/07

(54) **BIOMARKER BASED PATIENT SELECTION FOR PROTEASOME INHIBITOR TREATMENT**
BIOMARKERBASIERTE PATIENTENAUSWAHL ZUR PROTEASOMHEMMERBEHANDLUNG
SÉLECTION DE PATIENT À BASE DE BIOMARQUEUR DE TRAITEMENT PAR INHIBITEUR DE PROTÉASOME

(30) Priority: 17.06.2020 US 202063040442 P
(43) Date of publication of application: 26.04.2023
(73) Proprietor: University of Utah Research Foundation, Salt Lake City, UT 84108 (US)
(72) Inventor: WINTER, Jacob, M., Salt Lake City, UT 84112 (US); RUTTER, Jared, Salt Lake City, UT 84112 (US)
(74) Representative: CH Kilger Anwaltspartnerschaft mbB
(86) International application number: PCT/US2021/037943
(87) International publication number: WO 2021/257910

(56) References cited:
- WO-A1-2017/211947
- US-A1- 2015 301 053
- US-A1- 2016 346 401
- US-A1- 2016 346 401
- US-A1- 2017 327 895
- US-A1- 2017 327 895
- US-B2- 7 998 938
- POLURI RAGHAVENDRA TEJO KARTHIK ET AL: "Genomic Deletion at 10q23 in Prostate Cancer: More Than PTEN Loss?", FRONTIERS IN ONCOLOGY, vol. 8, 29 June 2018 (2018-06-29), XP055894290, ISSN: 2234-943X, DOI: 10.3389/fonc.2018.00246
- FLORIAN L. MULLER ET AL: "Collateral Lethality: A New Therapeutic Strategy in Oncology", SCIENTIFIC REPORTS, vol. 1, no. 3, 1 November 2015 (2015-11-01), US, pages 161 - 173, XP055271760, DOI: 10.1016/j.trecan.2015.10.002
- TEJO ET AL.: "Genomic Deletion at 10q23 in Prostate cancer: More than PTEN Loss?", FRONTIERS IN ONCOLOGY, vol. 8, 29 June 2018 (2018-06-29), pages 246, XP055894290, DOI: 10.3389/fonc.2018.00246

## Description

### FIELD

The present invention relates to proteasome inhibitor for use in treating cancer in a subject.

### BACKGROUND

The tumor suppressor gene, PTEN, is one of the most commonly deleted genes in human cancers (1). PTEN deletions often encompass many kilobases (kb) of adjacent DNA on chromosome 10q23, including neighboring loci. One of the nearest genes to PTEN is ATAD1, which encodes a AAA+ ATPase involved in protein quality control on the outer mitochondrial membrane (OMM) (2-6). ATAD1 is essential for life in humans and mice and it has been conserved over the 1 billion years of evolution separating yeast and humans (6,7). Herein is described how co-deletion of ATAD1 with PTEN sensitizes cells to dysfunction of the ubiquitin proteasome system (UPS). ATAD1 directly extracts the pro-apoptotic protein BIM from the OMM, demonstrating how protein quality control interfaces with cell death in a clinically relevant setting.

US2017/327895A1 discloses methods for using biomarkers to identify cancer patients that are likely to respond to treatment. It discloses that cell lines carrying a gene mutation in the PTEN gene are sensitive to the combination treatment with histone deacetylase 6 (HDAC6) inhibitor alone or in combination with bortezomib.

US2016/346401A1 discloses proteasome inhibitors, fibroblast activation protein (FAP)-activated prodrugs of proteasome inhibitors including bortezomib, carfilzomib, MLN-2238 (active form of ixazomib) and MLN-9708 (same as ixazomib), and pharmaceutically acceptable salts thereof for use in the treatment of cancer.

Poluri et al.: "Genomic Deletion at 10q23 in Prostate cancer: More than PTEN Loss?", Frontiers in Oncology, vol. 8, 29 June 2018 (2018-06-29), Article 246 discusses the genes that are lost along with PTEN upon deletion of the 10q23 locus that may play a role in prostate cancer (PCa) development.

### BRIEF SUMMARY

The invention is defined by the appended claims. The references to methods of treatment by therapy of this description are to be interpreted as references to compounds, pharmaceutical compositions and medicaments of the present invention for use in those methods.

Disclosed are proteasome inhibitor for use in treating a subject having cancer, wherein the subject has a non-functional ATAD1 gene, wherein the proteasome inhibitor is bortezomib or carfilzomib.

### BRIEF DESCRIPTION OF THE DRAWINGS

The accompanying drawings, which are incorporated in and constitute a part of this specification, illustrate several embodiments of the disclosed method and compositions and together with the description, serve to explain the principles of the disclosed method and compositions.

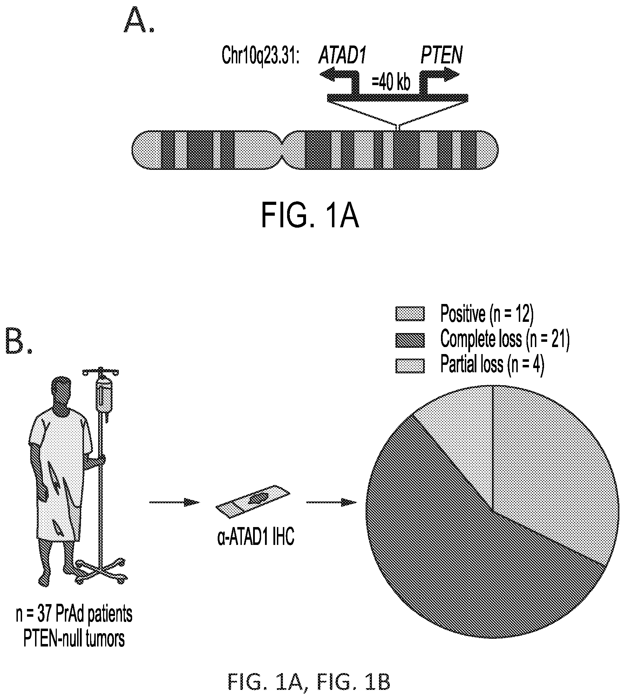

Figures 1A-1E show ATAD1 is frequently co-deleted with PTEN as a passenger gene in various cancers. (A) Schematic of ATAD1 and PTEN loci on human Chr10q23.31 (B) Assessing ATAD1 loss by IHC in patients with PTEN-null prostate cancer (C) Representative histology and IHC from two patients with PTEN-null tumors (D) TCGA Oncoprint showing co-deletion of ATAD1 and PTEN, and lack of mutations in ATAD1 alone (E) Frequency of alterations in ATAD1 across cancers, TCGA.

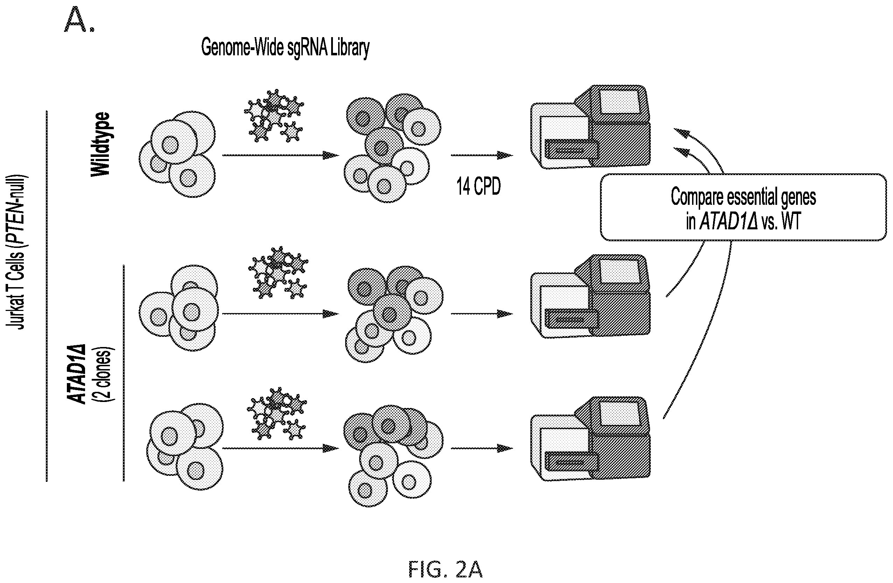

Figures 2A-2G show genome-wide CRISPR screens identify genetic interactions with ATAD1 (A) Schematic of CRISPR screening strategy (B) Generation of clonal *ATAD1* knockout in a *PTEN*-null genetic background of Jurkat cells and proliferation rate of *ATAD1*Δ and WT Jurkat cells in RPMI1640 media (C) Volcano plot of CRISPR screening results; CRISPR scores were defined as the mean Log₂ fold-change of sgRNAs targeting a given gene. Differential CRISPR scores (dCS) were calculated by subtracting the WT CS from the *ATAD1*Δ CS. dCS combined is the sum of dCS values for each clone. P-values were calculated for each gene using Kolmogorov-Smirnov with Benjamini Hochberg correction and Fisher's Method for P value meta-analysis. Dashed lines demarcate dCS < -2 and P_{adj} < 0.05 (D) DepMap data showing correlation of *MARCH5* dependency and *MCL1* dependency (E) Western blot of whole cell lysates from WT or *ATAD1*Δ Jurkat cells showing steady-state levels of BIM, MCL1, and FIS1, a non-BCL2 family control (F) Quantification of BIM_{EL} levels in ATAD1Δ cells; n = 6 independent experiments (G) Viability of WT or *ATAD1*Δ Jurkat cells after 24 hr treatment with the MCL1 inhibitor, AMG176.

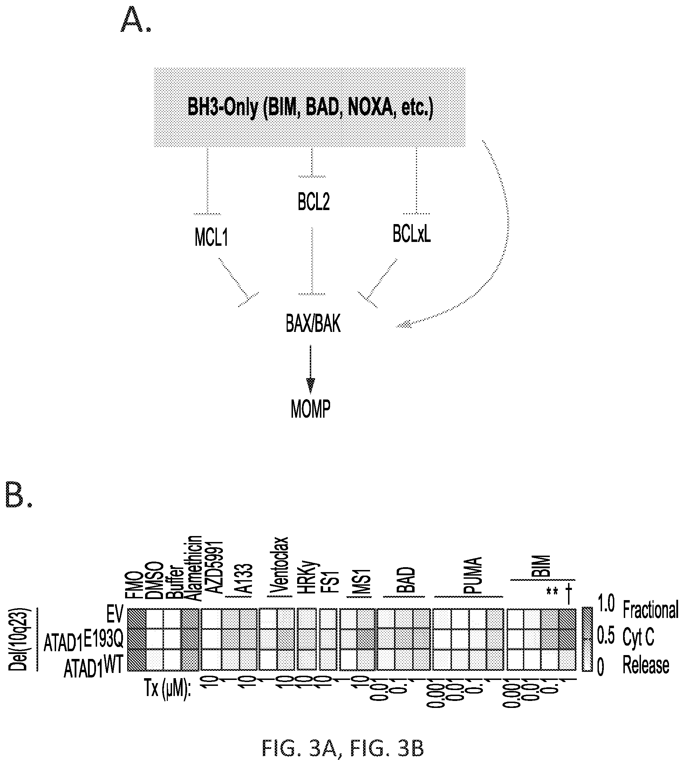

Figures 3A-3I show ATAD1 protects cells against BIM-mediated apoptotic priming by directly extracting BIM from membranes (A) Simplified schematic of BCL2-family control of mitochondrial outer membrane permeability (MOMP); See Fig S3A for binding preferences of BCL2-family proteins (B) BH3-profiling using Del10q23 cells (H4; PTEN-null, ATAD1-null) with EV, catalytically-dead ATAD1^{E193Q}, or wildtype ATAD1 stably re-expressed; heat map displays fractional cytochrome C release (MOMP) as the mean of 3 biological replicates (C) Viability of ATAD1-null or WT Jurkat cells stably expressing tetracycline-inducible GFP or GFP-BIM_{EL} fusion and treated with doxycycline for 48 hr (D) Apoptosis, as detected by PARP cleavage, in response to GFP-BIM_{EL} induction in ATAD1 wildtype or knockout cells (E) Western blot of GFP-BIM_{EL} protein in *ATAD1*Δ cells in a *BAK1* deletion background. Cells were treated with doxycycline (250 ng/mL) for 24 hr (F) Co-immunoprecipitation of ATAD1-FLAG/HA and GFP-BIM_{EL} in cells stably expressing ATAD1-FLAG or EV and transiently transfected overnight with GFP-BIM_{EL} in the presence of zVAD-fmk (G) RPMI7951 cells were treated with MG132 (10 µM) for 4 hr, whole-cell lysates were prepped and analyzed by Western blot. (H) Schematic of reconstituted proteoliposome extraction assay (I) Extraction assay using His-ATAD1 and 3xFLAG-BIM_{L} (lanes 1-4, 9-20) or the negative control TA protein, 3xFLAG-Fis1 (lanes 5-8); GST-tagged SGTA and calmodulin (CaM) are included as chaperones to catch extracted TA protein. "I" = Input, "FT" = flow-through, "W" = final wash, "E" = elution. Eluted fractions represent TA proteins extracted by ATAD1 and bound by GST-tagged chaperones; compare elution "E" to input "I". † denotes P < 0.0001

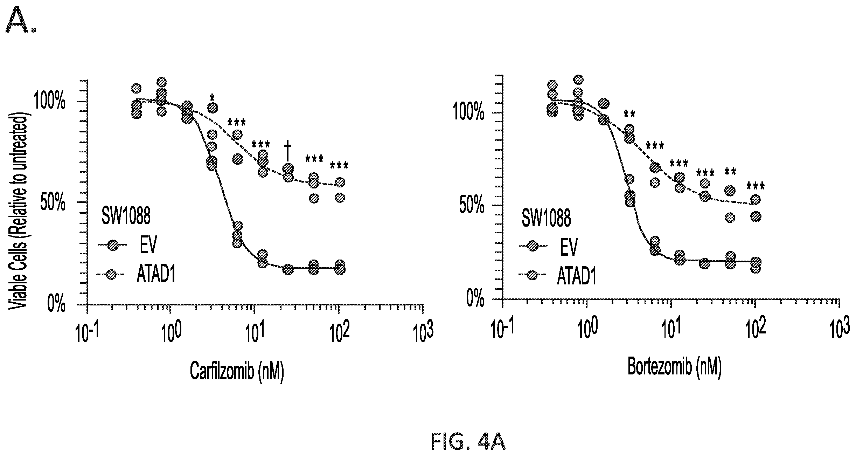

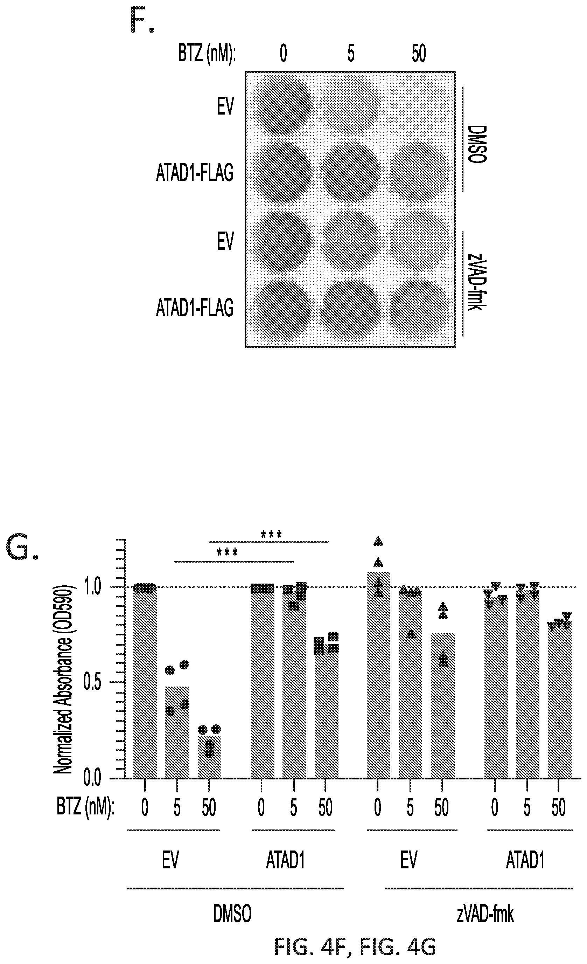

Figures 4A-4K show ATAD1 protects cells from apoptosis induced by proteasome dysfunction. (A) Viability of SW1088 cells transduced with EV or ATAD1-FLAG and treated for 24 hr with indicated drugs (B) Time-course of viability in response to bortezomib treatment in SW1088 cells (C) Western blot of RPMI7951 whole cell lysate treated with 10 nM bortezomib for 8 hr (D) Quantification of C (E) Viability of ATAD1-null vs. re-expressing RPMI7951 cells in response to 16 hr treatment with BTZ and the pan-caspase inhibitor, zVAD-FMK (20 µM) or DMSO (zVAD vehicle) (F) Same as E, but viability was measured by crystal violet staining and quantified in (G). (H) Schematic describing the role of ATAD1 in proteasome inhibitor toxicity (I) Tumor size of subcutaneous xenografts of SW1088 cells in NOD/SCID mice over time (J) Incidence of palpable tumors, or tumor-free survival, over time (K) Proliferation of cultured SW1088 cells as measured with crystal violet staining, n = 2 independent experiments. † represents P < 0.0001

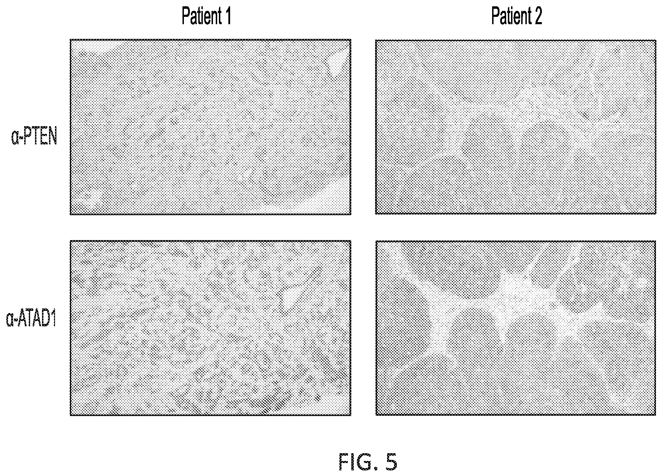

Figure 5 shows ATAD1 and PTEN IHC was conducted on biopsies from 15 patients with PTEN-positive tumors, and ATAD1 was detectable in all (PTEN-positive) samples.

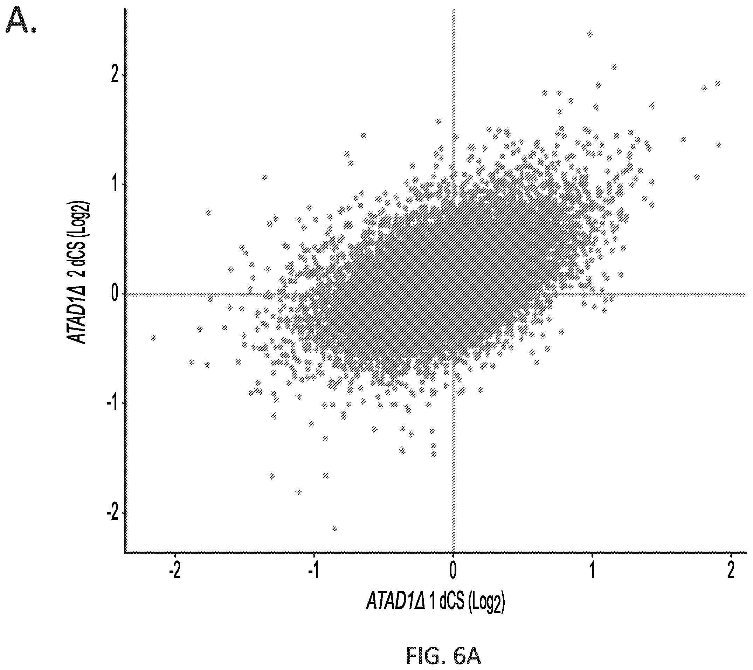

Figures 6A-6C show genome-wide CRISPR screens for genetic interactions with ATAD1. (A) Differential CRISPR Score for each clonal cell line vs. WT, plotted against each other. Pearson coefficient = 0.51, P = 2.16 x 10-16. (B) sgRNA-level data for differential fold change of each sgRNA targeting MARCH5 (C) Same as B for ACOT11

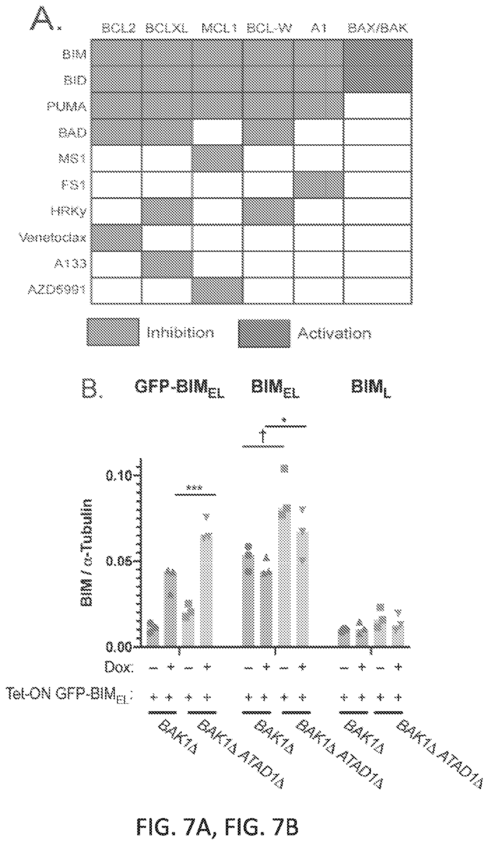

Figures 7A-7E show (A) Binding patterns of BH3 peptides and BH3 mimetic small molecules, related to BH3-profiling experiment in Figure 3B; derived from Letai, Ann Rev Cancer 2017, and Kale et al, Cell Death and Diff, 2018 (B) Quantification of Western blots of BIM isoforms in whole cell lysates of BAK1Δ and BAK1Δ ATAD1Δ cell lines stably expressing Tet-ON GFP-BIMEL. "GFP-BIMEL" indicates ectopic fusion protein, while "BIMEL" and "BIML" are endogenous. Cells were treated with 250 ng/mL doxycycline for 24 hr prior to harvest. n = 3 independent experiments (C) Soluble His-Msp1 and full-length Msp1 (D) extract BIML¬ from proteoliposomes. (E) The extraction assay recapitulates physiologic substrate selectivity of Msp1. Fis1 is extracted when a hydrophobic patch of residues is inserted N terminal to the TMD ("Fis1 - patch"). Sec22 and Sec61b are positive controls demonstrating that Msp1 can recognize ER-native TA proteins.

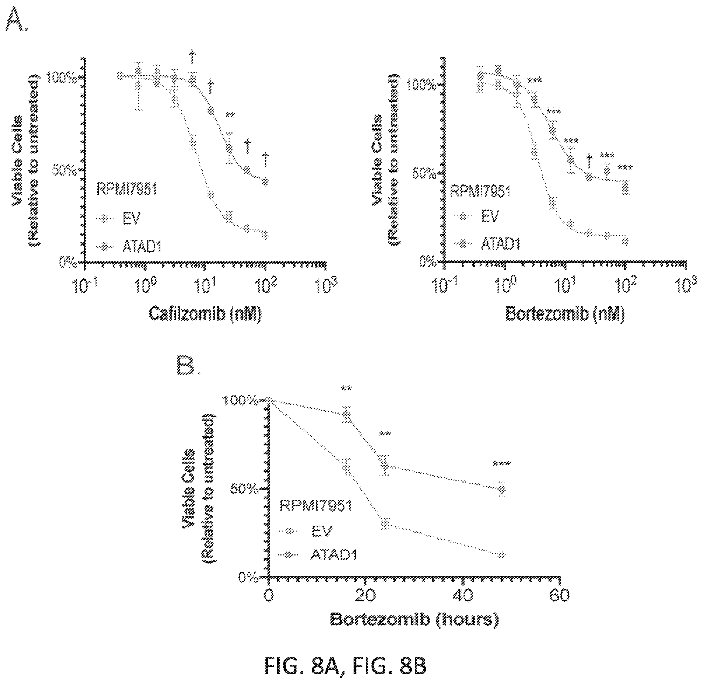

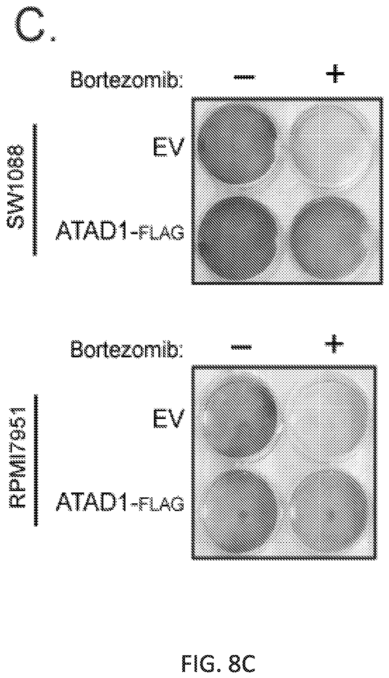

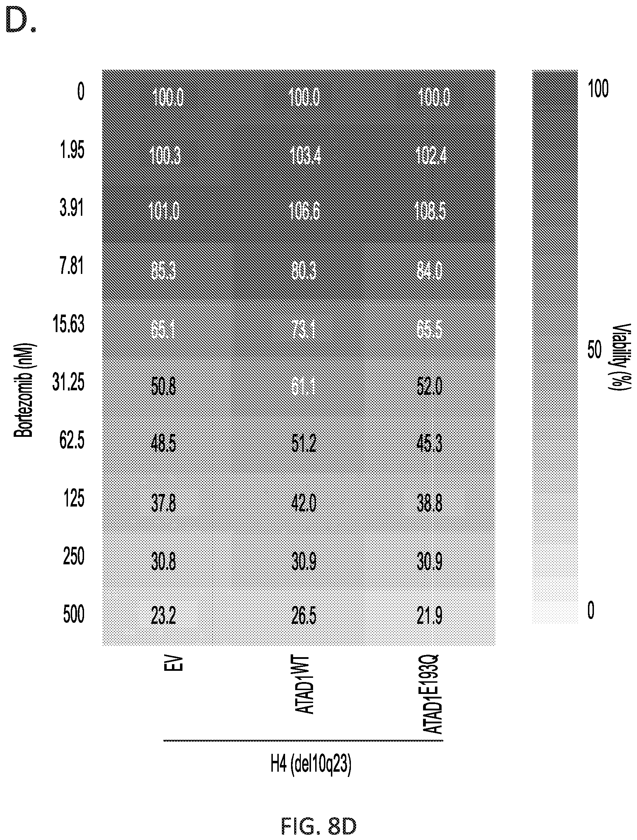

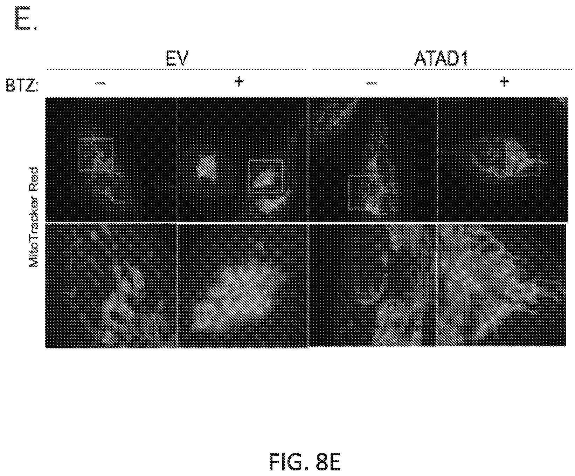

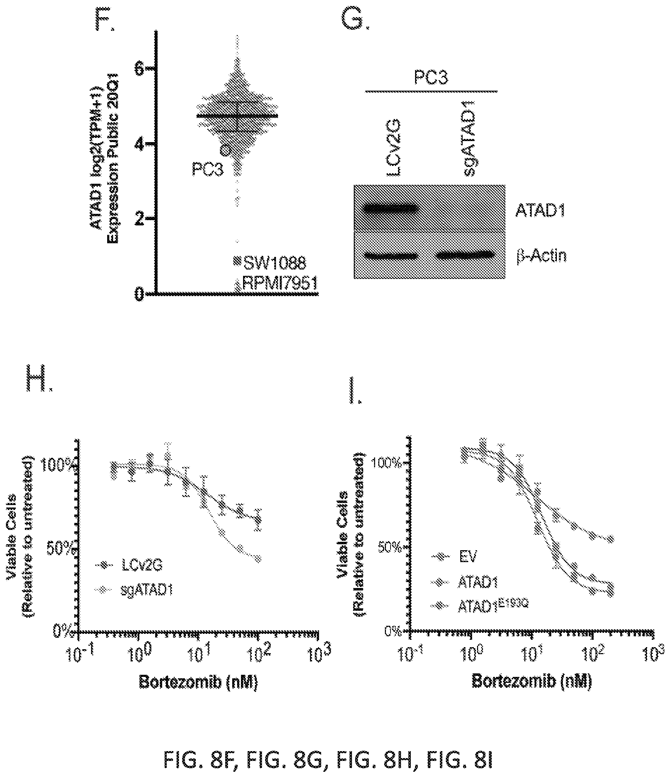

Figures 8A-8I show restoring ATAD1 expression in Del(10q23) cells increases resistance to proteasome inhibitors. (A) RPMI7951 cells were treated for 16 hr with bortezomib or carfilzomib and viability was measured by Cell Titer Glo. (B) Time-course of RPMI7951 cells treated with bortezomib (3.125 nM) n = 3 biological replicates (C) SW1088 cells were cultured in 5 nM bortezomib for 48 hr and RPMI7951 cells were cultured for 24 hr and viability was assessed by crystal violet staining (D) H4 cells were treated with bortezomib for 24 hr and assessed by Cell Titer Glo (E) ATAD1 addback rescues mitochondrial morphology phenotype in response to bortezomib in SW1088 cells. Cells were treated with BTZ (10 nM) for 12 hr and stained with MitoTracker Red, then imaged on a widefield fluorescence microscope (F) Expression of ATAD1 in DepMap cell lines, highlighting PC3 cells (ATAD1 hemizygous), and two Del10q23 cell lines (G) Generation of ATAD1-deficient, polyclonal PC3 cells using stable expression of sgATAD1 (in LCv2G), with untargeted Cas9 only as a control (LCv2G) (H) Deletion of ATAD1 increases sensitivity to bortezomib in PC3 cells. (I) Overexpression of ATAD1WT, but not ATAD1E193Q, increases resistance to bortezomib.

### DETAILED DESCRIPTION

The disclosed proteasome inhibitor may be understood more readily by reference to the following detailed description of particular embodiments and the Example included therein and to the Figures and their previous and following description.

### A. Definitions

It must be noted that as used herein and in the appended claims, the singular forms "a ", "an", and "the" include plural reference unless the context clearly dictates otherwise. Thus, for example, reference to "a subject" includes a plurality of such subjects, reference to "the proteasome inhibitor" is a reference to one or more proteasome inhibitors and equivalents thereof known to those skilled in the art, and so forth.

As used herein, the term "subject" or "patient" can be used interchangeably and refer to any organism to which a composition of this invention may be administered, e.g., for experimental, diagnostic, and/or therapeutic purposes. Typical subjects include animals (e.g., mammals such as non-human primates, and humans; avians; domestic household or farm animals such as cats, dogs, sheep, goats, cattle, horses and pigs; laboratory animals such as mice, rats and guinea pigs; rabbits; fish; reptiles; zoo and wild animals). Typically, "subjects" are animals, including mammals such as humans and primates; and the like.

By "treat" is meant to administer a therapeutic, such as a proteasome inhibitor, to a subject, such as a human or other mammal (for example, an animal model), that has an increased susceptibility for developing cancer, in order to prevent or delay a worsening of the effects of the disease or condition, or to partially or fully reverse the effects of the disease or condition (e.g. cancer).

By "prevent" is meant to minimize the chance that a subject who has an increased susceptibility for developing cancer.

As used herein, the terms "administering" and "administration" refer to any method of providing a therapeutic, such as a proteasome inhibitor, to a subject. Such methods are well known to those skilled in the art and include, but are not limited to: oral administration, transdermal administration, administration by inhalation, nasal administration, topical administration, intravaginal administration, ophthalmic administration, intraaural administration, intracerebral administration, rectal administration, sublingual administration, buccal administration, and parenteral administration, including injectable such as intravenous administration, intra-arterial administration, intramuscular administration, and subcutaneous administration. Administration can be continuous or intermittent. In various aspects, a preparation can be administered therapeutically; that is, administered to treat an existing disease or condition. In further various aspects, a preparation can be administered prophylactically; that is, administered for prevention of a disease or condition. In an aspect, the skilled person can determine an efficacious dose, an efficacious schedule, or an efficacious route of administration so as to treat a subject.

As used herein, "biological sample" refers to any sample that can be from or derived from a mammal, particularly a human patient, e.g., bodily fluids (blood, saliva, urine etc.), biopsy, tissue, and/or waste from the patient. Thus, tissue biopsies, stool, sputum, saliva, blood, plasma, serum, lymph, tears, sweat, urine, vaginal secretions, or the like can easily be screened for SNPs, as can essentially any tissue of interest that contains the appropriate nucleic acids. These samples are typically taken, following informed consent, from a patient by standard medical laboratory methods. The sample may be in a form taken directly from the patient, or may be at least partially processed (purified) to remove at least some non-nucleic acid material.

As used herein, "modulate" is meant to mean to alter, by increasing or decreasing. Modulate can mean a change in activity or function or number. The change can be an increase or decrease, an enhancement or an inhibition of activity, function or number.

Ranges may be expressed herein as from "about" one particular value, and/or to "about" another particular value. When such a range is expressed, also specifically contemplated and considered disclosed is the range from the one particular value and/or to the other particular value unless the context specifically indicates otherwise. Similarly, when values are expressed as approximations, by use of the antecedent "about," it will be understood that the particular value forms another, specifically contemplated embodiment that should be considered disclosed unless the context specifically indicates otherwise. It will be further understood that the endpoints of each of the ranges are significant both in relation to the other endpoint, and independently of the other endpoint unless the context specifically indicates otherwise. Finally, it should be understood that all of the individual values and sub-ranges of values contained within an explicitly disclosed range are also specifically contemplated and should be considered disclosed unless the context specifically indicates otherwise. The foregoing applies regardless of whether in particular cases some or all of these embodiments are explicitly disclosed.

Unless defined otherwise, all technical and scientific terms used herein have the same meanings as commonly understood by one of skill in the art to which the disclosed method and compositions belong. Although any methods and materials similar or equivalent to those described herein can be used in the practice or testing of the present method and compositions, the particularly useful methods, devices, and materials are as described.

Throughout the description and claims of this specification, the word "comprise" and variations of the word, such as "comprising" and "comprises," means "including but not limited to," and is not intended to exclude, for example, other additives, components, integers or steps. In particular, in methods stated as comprising one or more steps or operations it is specifically contemplated that each step comprises what is listed (unless that step includes a limiting term such as "consisting of"), meaning that each step is not intended to exclude, for example, other additives, components, integers or steps that are not listed in the step.

### B. Compounds for use in treating

Disclosed is a proteasome inhibitor for use in treating cancer in a subject, wherein the subject has a non-functional ATAD1 gene; and wherein the proteasome inhibitor is bortezomib or carfilzomib.

In some aspects, a non-functional ATAD1 gene can be a deleted gene or a mutated gene. In some aspects, a non-functional ATAD1 gene can be partially or completely deleted.

In some aspects, the subject further comprises a non-functional PTEN. In some aspects, a non-functional PTEN can be a non-functional PTEN gene or a non-functional PTEN protein. A non-functional PTEN gene can be a deleted gene or a mutated gene. In some aspects, a non-functional PTEN gene can be partially or completely deleted. In some aspects, non-functional PTEN protein can be a protein translated from a non-functional PTEN gene. In some aspects, non-functional PTEN protein can be translated into a functional protein and then post-translationally modified into a non-functional PTEN protein.

In some aspects, the subject having cancer has breast cancer, lung cancer, colon cancer, brain cancer, or prostate cancer.

The proteasome inhibitor is bortezomib or carfilzomib.

### C. Compound for use in Increasing Apoptosis

Disclosed is a proteasome inhibitor for use in treating cancer in a subject by increasing apoptosis in said subject, wherein the subject comprises a non-functional ATAD1 gene; and wherein the proteasome inhibitor is bortezomib or carfilzomib. A non-functional ATAD1 gene can be a deleted gene or a mutated gene. In some aspects, a non-functional ATAD1 gene can be partially or completely deleted.

In some aspects, the subject further comprises a non-functional PTEN. In some aspects, a non-functional PTEN can be a non-functional PTEN gene or a non-functional PTEN protein. A non-functional PTEN gene can be a deleted gene or a mutated gene. In some aspects, a non-functional PTEN gene can be partially or completely deleted. In some aspects, non-functional PTEN protein can be a protein translated from a non-functional PTEN gene. In some aspects, non-functional PTEN protein can be translated into a functional protein and then post-translationally modified into a non-functional PTEN protein.

In some aspects, the subject has cancer. In some aspects, the subject has breast cancer, lung cancer, colon cancer, brain cancer, or prostate cancer.

The proteasome inhibitor is bortezomib or carfilzomib.

### D. Methods of Identifying a Subject (not part of the claimed invention)

Disclosed, but nor part of the claimed invention; are methods comprising the steps in the following order obtaining a sample from a cancer patient; determining whether the cancer patient has a non-functional ATAD1 in the sample; identifying the cancer patient as a suitable candidate for treatment with a proteasome inhibitor if the cancer patient has a non-functional ATAD1; and administering a proteasome inhibitor to the cancer patient identified as the suitable candidate, and not administering a proteasome inhibitor to the cancer patient if the cancer patient does not have a non-functional ATAD1.

Disclosed are methods comprising the steps in the following order determining whether a cancer patient has a non-functional ATAD1 in a sample obtained from the cancer patient; identifying the cancer patient as a suitable candidate for treatment with a proteasome inhibitor if the cancer patient has a non-functional ATAD1; and administering a proteasome inhibitor to the cancer patient identified as the suitable candidate, and not administering a proteasome inhibitor to the cancer patient if the cancer patient does not have a non-functional ATAD1.

Disclosed are methods comprising the steps in the following order obtaining a sample from a cancer patient; determining whether the cancer patient has a non-functional ATAD1 in the sample; identifying the cancer patient as a suitable candidate for treatment with a compound or therapeutic that modulates protein quality control of protein degradation if the cancer patient has a non-functional ATAD1; and administering a compound or therapeutic that modulates protein quality control of protein degradation to the cancer patient identified as the suitable candidate, and not administering a compound or therapeutic that modulates protein quality control of protein degradation to the cancer patient if the cancer patient does not have a non-functional ATAD1.

Disclosed are methods comprising the steps in the following order determining whether a cancer patient has a non-functional ATAD1 in a sample obtained from the cancer patient; identifying the cancer patient as a suitable candidate for treatment with a compound or therapeutic that modulates protein quality control of protein degradation if the subject has a non-functional ATAD1; and administering a compound or therapeutic that modulates protein quality control of protein degradation to the cancer patient identified as the suitable candidate, and not administering a compound or therapeutic that modulates protein quality control of protein degradation to the cancer patient if the cancer patient does not have a non-functional ATAD1.

In some aspects, a non-functional ATAD1 gene can be a deleted gene or a mutated gene. In some aspects, a non-functional ATAD1 gene can be partially or completely deleted. In some aspects, non-functional ATAD1 protein can be a protein translated from a non-functional ATAD1 gene. In some aspects, non-functional ATAD1 protein can be translated into a functional protein and then post-translationally modified into a non-functional ATAD1 protein.

In some aspects, the method further comprises determining whether the cancer patient has a non-functional PTEN. In some aspects, a non-functional PTEN can be a non-functional PTEN gene or a non-functional PTEN protein. A non-functional PTEN gene can be a deleted gene or a mutated gene. In some aspects, a non-functional PTEN gene can be partially or completely deleted. In some aspects, non-functional PTEN protein can be a protein translated from a non-functional PTEN gene. In some aspects, non-functional PTEN protein can be translated into a functional protein and then post-translationally modified into a non-functional PTEN protein.

Also disclosed are methods of enhancing the efficacy of a proteasome inhibitor in a subject with cancer, the method comprising administering to the subject an effective amount of a proteasome inhibitor to a subject with a non-functional ATAD1. Also disclosed are methods of enhancing the efficacy of a compound or therapeutic that modulates protein quality control of protein degradation in a subject with cancer, the method comprising administering to the subject an effective amount of a compound or therapeutic that modulates protein quality control of protein degradation to a subject with a non-functional ATAD1. In some aspects, a non-functional ATAD1 gene can be a deleted gene or a mutated gene. In some aspects, a non-functional ATAD1 gene can be partially or completely deleted. In some aspects, non-functional ATAD1 protein can be a protein translated from a non-functional ATAD1 gene. In some aspects, non-functional ATAD1 protein can be translated into a functional protein and then post-translationally modified into a non-functional ATAD1 protein. In some aspects, the subject further comprises a non-functional PTEN. In some aspects, a non-functional PTEN can be a non-functional PTEN gene or a non-functional PTEN protein. A non-functional PTEN gene can be a deleted gene or a mutated gene. In some aspects, a non-functional PTEN gene can be partially or completely deleted. In some aspects, non-functional PTEN protein can be a protein translated from a non-functional PTEN gene. In some aspects, non-functional PTEN protein can be translated into a functional protein and then post-translationally modified into a non-functional PTEN protein.

In some aspects, the cancer patient has breast cancer, lung cancer, colon cancer, brain cancer, or prostate cancer.

Described herein are methods of identifying a subject appropriate for a clinical trial comprising determining in the subject the presence of a non-functional ATAD1 gene, wherein the presence of a non-functional ATAD1 indicates the subject is appropriate for a clinical trial for treatment with a proteasome inhibitor. Being "appropriate for a clinical trial" refers to a subject that could likely benefit from the treatment of the clinical trial. For example, a subject identified as having a non-functional ATAD1 can be considered in need of treatment, responsive or susceptible to treatment with a proteasome inhibitor and therefore is appropriate for a clinical trial for a proteasome inhibitor. For example, described herein are methods of identifying a subject appropriate for a clinical trial with a proteasome inhibitor comprising determining in the subject the presence of a non-functional ATAD1, wherein the presence of a non-functional ATAD1 indicates the subject is appropriate for a clinical trial for a proteasome inhibitor.

Also described herein are methods of enhancing clinical trials comprising choosing appropriate patient populations for those clinical trials. In one aspect, the methods can be used to ensure patients are identified to participate in clinical trials based upon the likelihood of whether subject can benefit from treatment with a proteasome inhibitor or a compound or therapeutic that modulates protein quality control of protein degradation. In some aspects, the methods comprises determining whether the subject has a non-functional ATAD1 thereby identifying a subject that can benefit from treatment with a proteasome inhibitor or a compound or therapeutic that modulates protein quality control of protein degradation.

Disclosed herein are methods for identifying a subject for a clinical study, the method comprising detecting in a sample from the subject a non-functional ATAD1 gene and wherein the presence a non-functional ATAD1 indicate the subject is appropriate for the clinical trial, wherein the non-functional ATAD1 in the subject is determined from a sample obtained from the subject.

In some aspects, the proteasome inhibitor is selected from the group of bortezomib, ixazomib, carfilzomib, Lactacystin, Disulfiram, Epigallocatechin-3-gallate, Marizomib (salinosporamide A), Oprozomib, delanzomib, Epoxomicin, MG132, Beta-hydroxy beta-methylbutyrate.

In some aspects, a compound or therapeutic that modulates protein quality control of protein degradation can be, but is not limited to, IRE1 modulators, PERK modulators, ATF6 modulators, heat shock protein inhibitors, selective androgen receptor degraders, or selective estrogen receptor degraders.

### Examples

### A. Example 1

The tumor suppressor gene, PTEN, is one of the most commonly deleted genes in human cancers (1). PTEN deletions often encompass many kilobases (kb) of adjacent DNA on chromosome 10q23, including neighboring loci. One of the nearest genes to PTEN is ATAD1, which encodes a AAA+ ATPase involved in protein quality control on the outer mitochondrial membrane (OMM) (2-6). ATAD1 is essential for life in humans and mice and it has been conserved over the 1 billion years of evolution separating yeast and humans (6,7). Herein is described how co-deletion of ATAD1 with PTEN sensitizes cells to dysfunction of the ubiquitin proteasome system (UPS). ATAD1 directly extracts the pro-apoptotic protein BIM from the OMM, demonstrating how protein quality control interfaces with cell death in a clinically relevant setting.

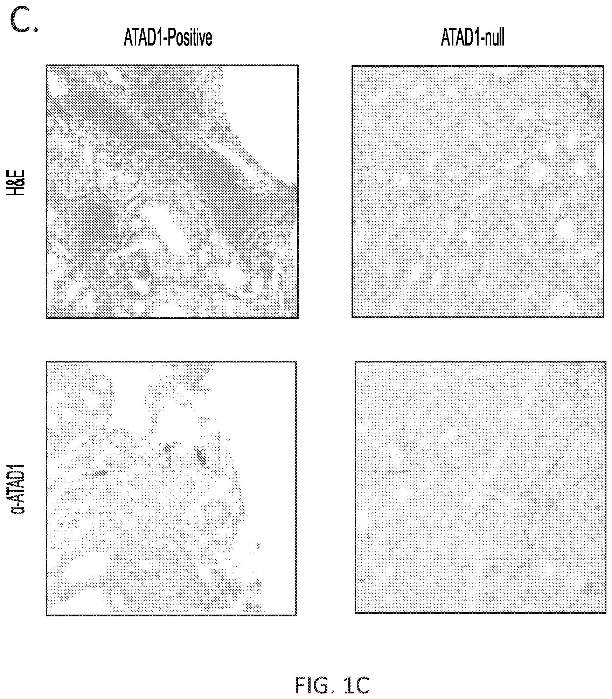

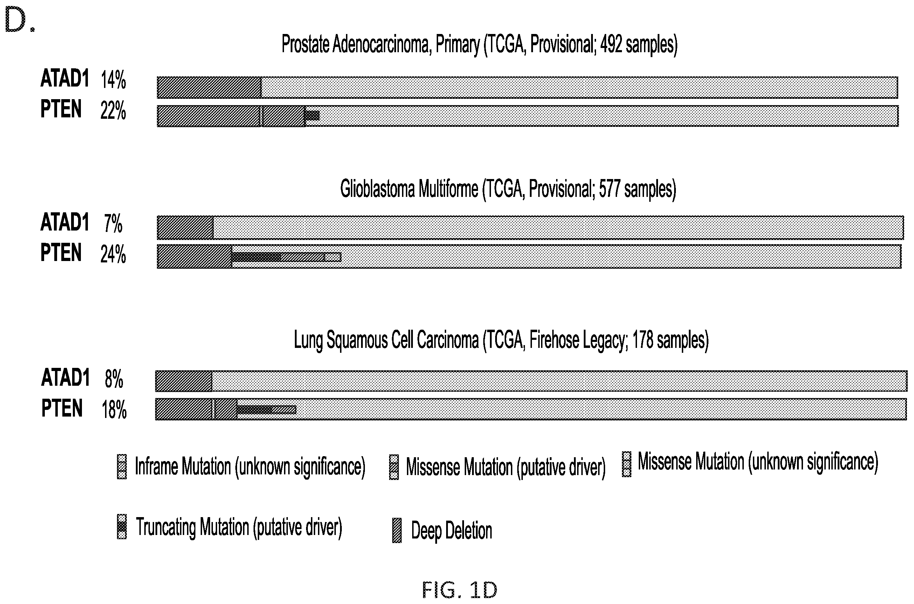

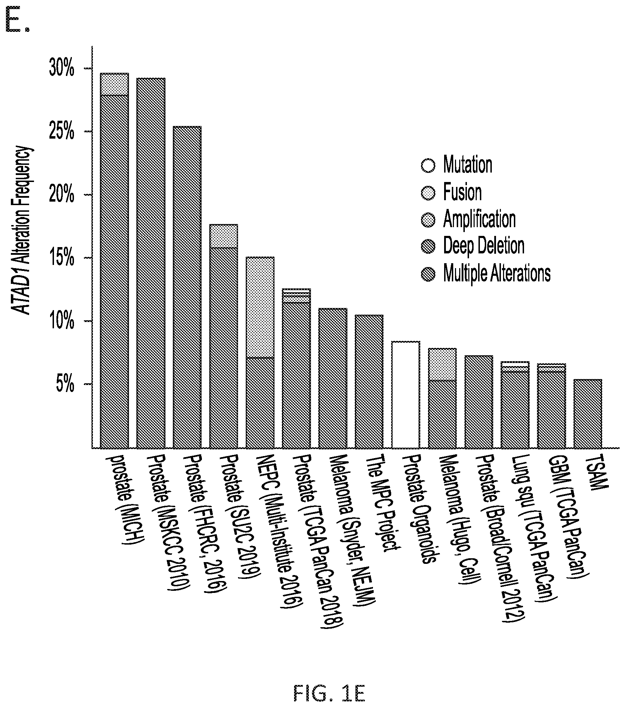

The PTEN and ATAD1 loci are separated by approximately 40 kb on human Chr10q23.31 (Fig 1A) (8). Due to this proximity, whether ATAD1 is co-deleted with PTEN is assessed using immunohistochemistry on tumor specimens from patients with prostate adenocarcinoma (PrAd), which is known to feature PTEN deletion (9). ATAD1 status is assessed using samples from 37 patients who had PTEN-null tumors and found that ATAD1 was undetectable in more than half (21/37), but was retained in all 15 PTEN-positive control specimens (Fig 1B,C, Fig 5). To corroborate the protein-level findings, The Cancer Genome Atlas (TCGA) was analyzed. The majority of tumors harboring deep deletions in PTEN also had deep deletions in ATAD1 (Fig 1D), and this held true across multiple types of cancer (Fig 1E) (8). Importantly, TCGA data also showed that ATAD1 is almost never deleted in the absence of PTEN deletion (Fig 1D), and there are essentially no inactivating point mutations or truncations in ATAD1, unlike those seen in PTEN and most bona fide tumor suppressors. Therefore, ATAD1 deletion is solely a passenger to the oncogenic driver of PTEN deletion. (Fig 1D). Loss of ATAD1 occurs at a high frequency across cancers, including in up to more than 25% of PrAd, 11% of melanoma, and 7-8% of glioblastoma and lung squamous cell carcinoma. Therefore, understanding how loss of ATAD1 affects these tumors could have a profound impact in the clinic.

ATAD1 and its homologs, including the S. cerevisiae homolog Msp1, protect mitochondrial integrity by extracting mislocalized or excess transmembrane proteins from the OMM and peroxisomes (10). Msp1 has also been shown to extract proteins stalled in the mitochondrial import channel, the TOM complex (11,12). Given the established role of ATAD1 in mitochondrial homeostasis in other contexts, deletion of ATAD1 as a passenger gene can confer unique vulnerabilities on tumors. Such vulnerabilities could represent novel therapeutic targets for precision medicine, a concept known as "collateral lethality" of tumor suppressor gene deletion (13,14).

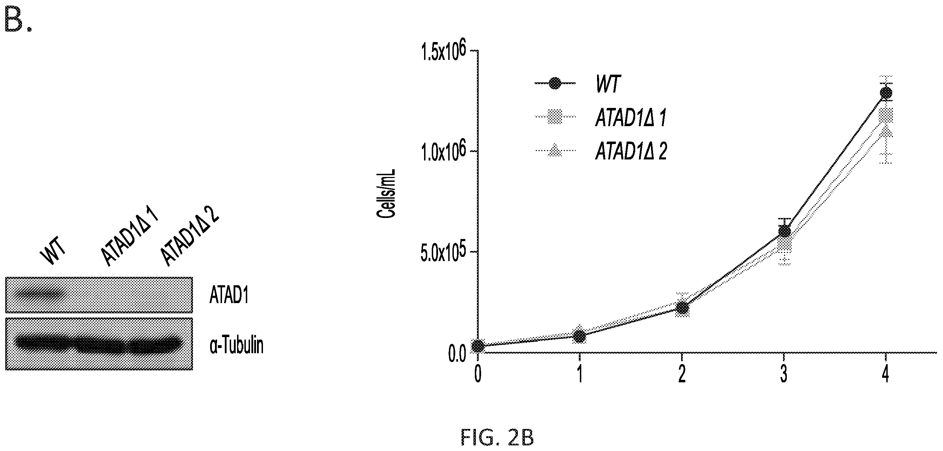

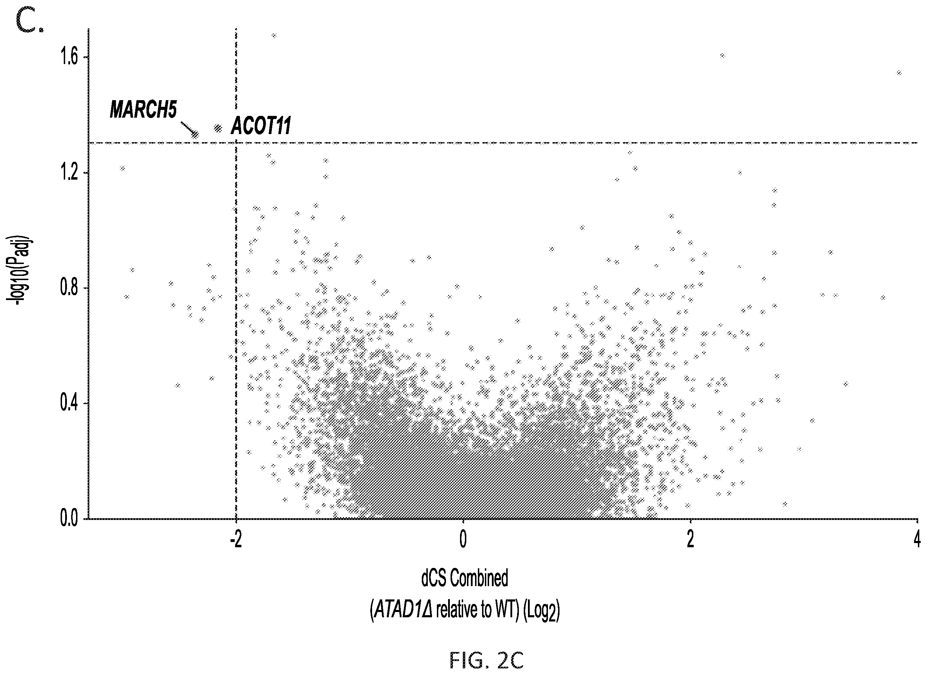

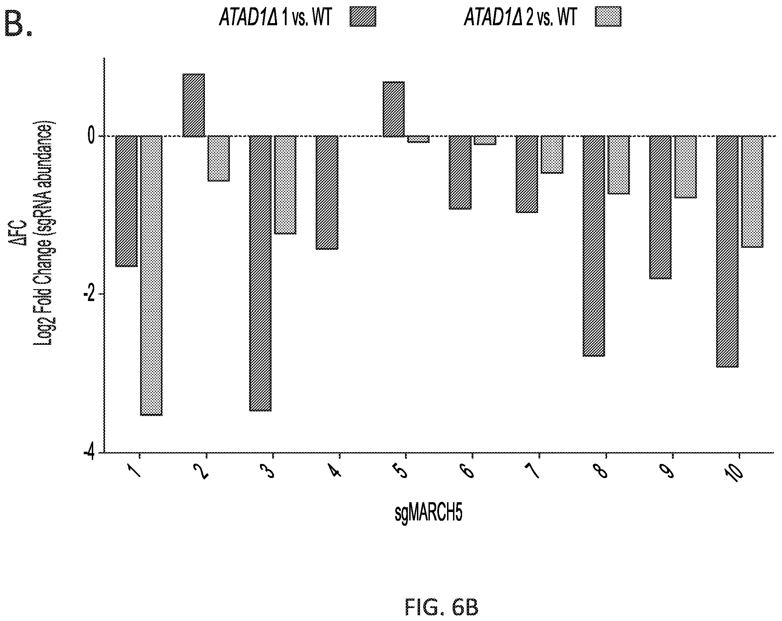

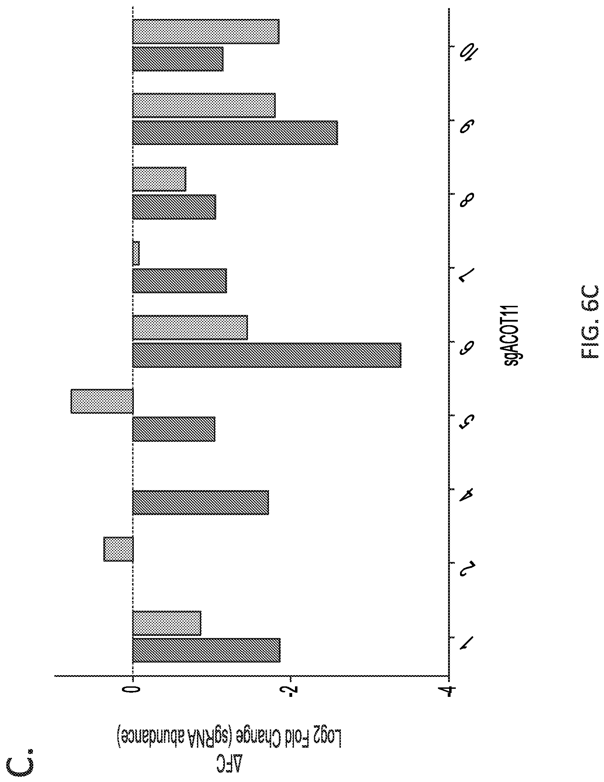

To discover such vulnerabilities, genome-wide CRISPR screens were conducted to identify genes that are selectively essential in ATAD1Δ cells. Two clonal ATAD1Δ lines were generated in Jurkat cells, which are PTEN-null, using transient expression of Cas9 and two distinct sgRNA targeting ATAD1 (Fig 2A). ATAD1 deletion did not affect basal proliferation rate (Fig 2B). Three screens were conducted in parallel on the wildtype (WT) Jurkat parental cell line and each of the two ATAD1Δ clonal cell lines, the comparison of which enabled us to eliminate idiosyncrasies inherent to clonal cell lines (Fig 2C). Differential CRISPR Scores (dCS) represent the difference between WT and ATAD1Δ in Log2 fold-change of mean abundance of sgRNAs targeting a given gene, providing a metric for whether the deletion of that gene is more or less detrimental to cell fitness in the absence of ATAD1. As expected, dCS values for the two clonal cell lines significantly correlated (Fig. 6A). P values were combined to penalize genes that scored as hits in only one of the two ATAD1Δ clones (15). Two genes emerged as hits, as defined by a combined dCS value of < -2 and Padj < 0.05: ACOT11 and MARCH5 (Fig 2C, Fig 6B,C). ACOT11 encodes an acyl-CoA thioesterase that localizes to mitochondria and the cytoplasm and hydrolyzes long-chain fatty acyl-CoAs (16,17). MARCH5 encodes a ubiquitin E3 ligase localized to the OMM that targets a number of transmembrane proteins for degradation (18-20).

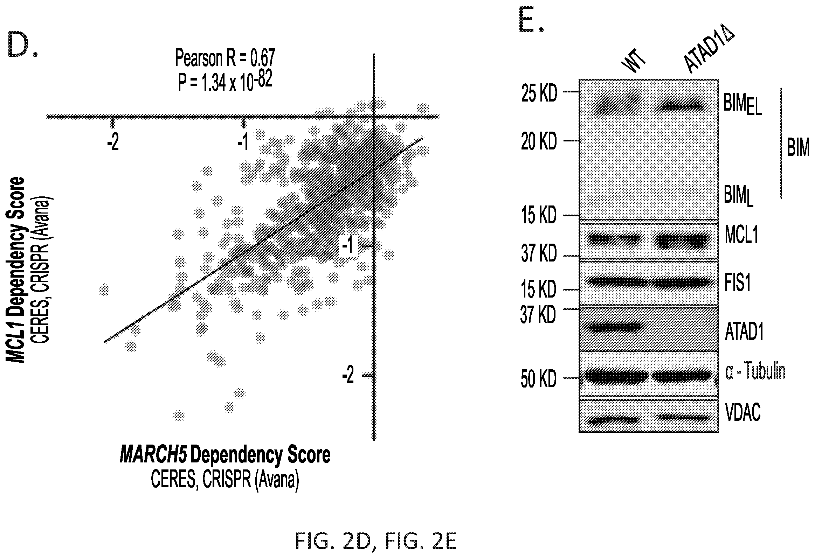

MARCH5 was recently shown to promote degradation of members of the BCL2 family, which is comprised of pro and anti-apoptotic proteins that function at the OMM to regulate the initiation of apoptosis (21-24). The critical step in the regulation of apoptosis is the permeabilization of the OMM by BAX/BAK, which causes mitochondrial proteins such as cytochrome C to diffuse into the cytoplasm and activate the caspase cascade. Anti-apoptotic proteins, including BCL2, BCL-XL, and MCL1, bind to and inhibit BAX and BAK under basal conditions (23). Upstream of anti-apoptotic proteins are the pro-death BH3-only proteins (including BIM, BID, NOXA, BAD, PUMA, etc.), which respond to stress by inactivating specific anti-apoptotic proteins or directly activating BAX/BAK (25). Deletion of MARCH5 causes MCL1, NOXA, and BIM to accumulate on the OMM, and increases sensitivity to BH3 mimetic drugs, which bind to and sequester anti-apoptotic BCL2 family members to trigger apoptosis (26-29). As noted by others, MARCH5 and MCL1 (encoding a potent anti-apoptotic protein) are co-essential (Fig 2D), which indicates that they function in the same pathway (29,30). In fact, according to the DepMap database, the most co-essential gene with MCL1 across the entire human genome is MARCH5, and vice versa (31).

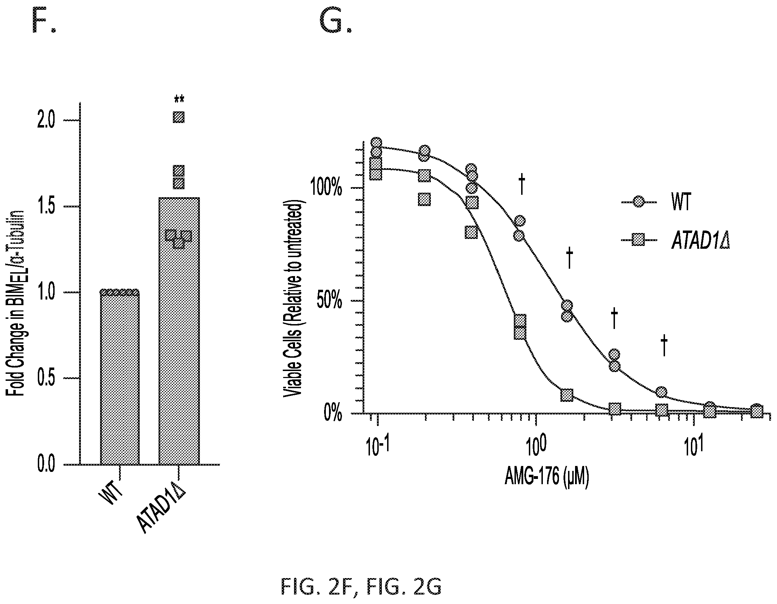

That ATAD1 and MARCH5 were synthetic lethal in the screen indicated that they could act in parallel pathways or functions. Indeed, although ATAD1 deletion had no effect on FIS1, a tail-anchored protein on the OMM that is not part of the BCL2 family, it increased the abundance of MCL1 and BIM (Fig 2E,F) and primed cells for apoptosis induced by AMG176, a BH3-mimetic that inhibits MCL1 (Fig 2G) (32). Thus, like MARCH5, ATAD1 modulates members the BCL2 family and affects apoptotic priming as measured by sensitivity to a BH3-mimetic.

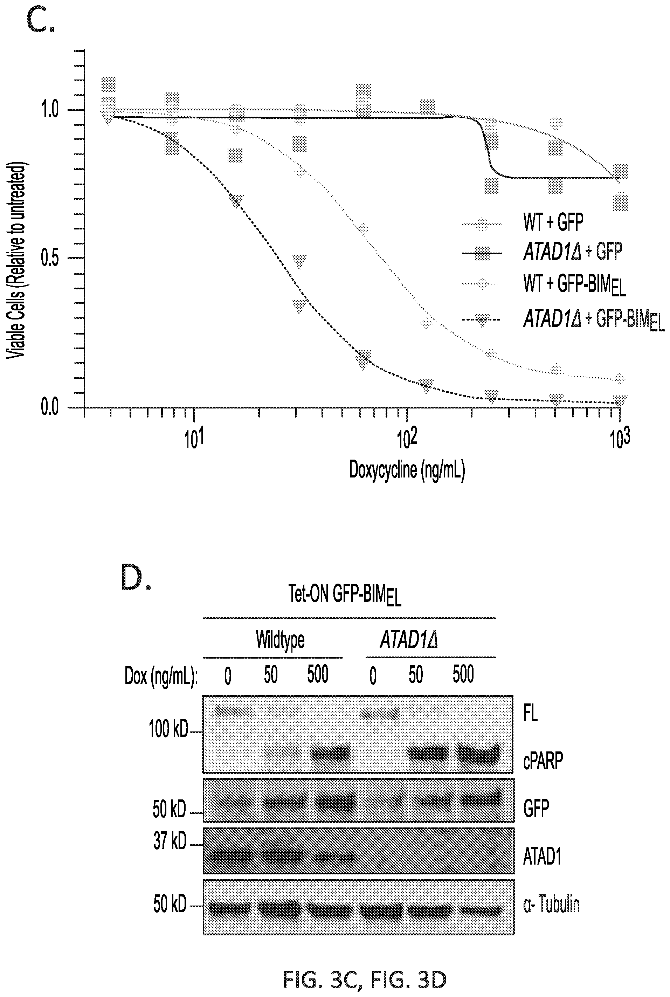

To gain insight into how loss of ATAD1 affects apoptotic priming, BH3 profiling was conducted(33). Here, permeabilized cells are treated with BH3 peptides derived from different pro-apoptotic BH3-only proteins, and mitochondrial outer membrane permeability (MOMP) is directly measured by detecting cytochrome C release from mitochondria (Fig 3A). H4 glioma cells were profiled, which harbor a Chr10q23 deletion encompassing both ATAD1 and PTEN, in which there was stably re-expressed ATAD1, a catalytically inactive mutant ATAD1E193Q, or the empty vector (EV). In these ATAD1-null cells expressing EV or ATAD1E193Q, BIM peptide caused massive release of cytochrome C, while expression of WT ATAD1 significantly protected cells from BIM-mediated cytochrome C release (Fig 3B). In contrast, PUMA and BAD induced moderate cytochrome C release independent of ATAD1 status. Alamethicin, a synthetic pore-forming peptide that operates independently of BAX/BAK and BH3-only proteins, induced cytochrome C release equally regardless of ATAD1 status, which indicates that ATAD1 does not affect cytochrome C release downstream of pore formation on the OMM. These data show that the absence of ATAD1 increases apoptotic priming that is sensitive to BIM peptide. This priming reflects a change in how the cell regulates the endogenous BIM protein. To determine if ATAD1 protected cells from full-length BIM protein in addition to BIM peptide, cell lines stably expressing tetracycline-inducible GFP-BIMEL fusion protein were generated. BIM exists in three main isoforms, BIMEL ("Extra-Long", the predominant form), BIML ("Long"), and BIMS ("Short"), which share key structural features including a C-terminal membrane anchor (34-36). Similar to the BH3-profiling experiment, it was found that loss of ATAD1 sensitized cells to ectopic, full-length GFP-BIM, as evidenced by increased cell death and generation of cleaved PARP (Fig 3C,D). Thus, the biochemical and genetic data indicate that ATAD1 deficiency sensitizes to BIM, a potent pro-apoptotic BH3-only protein.

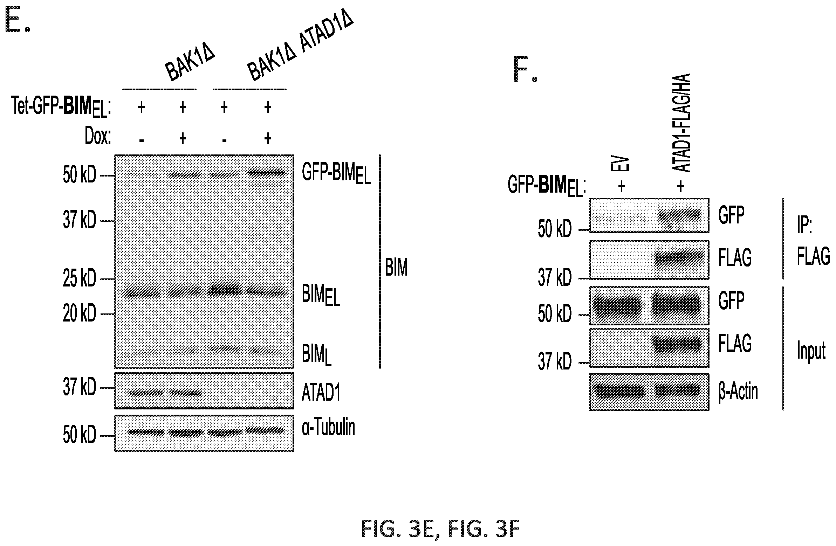

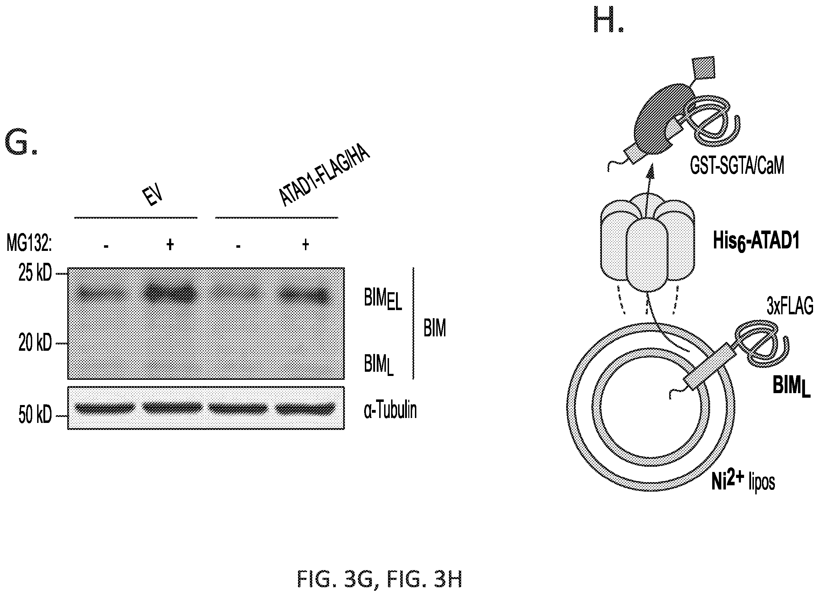

BIM can be a direct substrate of ATAD1 because BIM has some features of known ATAD1/Msp1 substrates: it is tail-anchored, intrinsically disordered, has basic residues C-terminal to its transmembrane domain, and is degraded by the proteasome (37-39). Deletion of ATAD1 caused modestly increased BIM abundance (Fig 2E,F) and what might be a change in BIM phosphorylation status, based on a shift in observed molecular weight (34,40). How ATAD1 affected BIM abundance was examined in cells challenged with ectopic BIM. This experiment was performed in BAK1Δ Jurkat cell lines (which are basally BAX-null) that are resistant to BIM-induced apoptosis to prevent cell death from confounding the results. In this context, loss of ATAD1 caused accumulation of ectopic GFP-BIMEL, endogenous BIMEL, and a trend toward accumulation of endogenous BIML (Fig 3E, Fig 7B). Consistent with BIM being an ATAD1 substrate, GFP-BIMEL co-immunoprecipitated with ATAD1-FLAG (Fig 3F). BIM can be degraded by the proteasome, and accordingly, it was found that acute proteasome inhibition caused a robust increase in BIM levels (Fig 3G) in both the presence and absence of ATAD1. Altogether, ATAD1 deficiency sensitizes cells to a BH3-mimetic, BIM peptide, and full-length BIM; BIM hyper-accumulates in ATAD1-null cells; and BIM physically interacts with ATAD1. These data provide a compelling case that BIM is an ATAD1 substrate in cells.

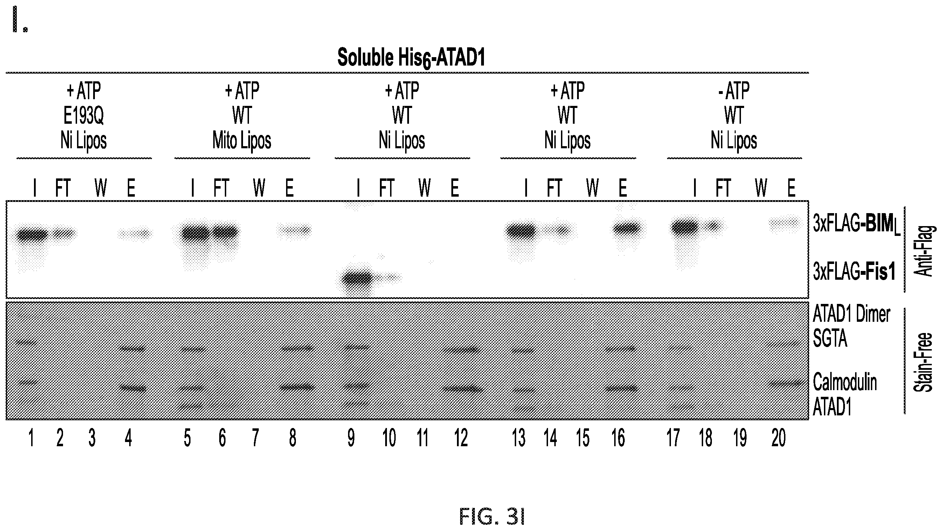

To address whether BIM is a direct substrate of ATAD1, an in vitro extraction assay was used with ATAD1 and BIML. BIML was used because it is more soluble than BIMEL but shares all key structural features except for a domain near the N terminus (34). full-length ATAD1 was not able to be purified. Instead, the N-terminal TMD was swapped with a His6 tag, which anchored His-ATAD1 to liposomes doped with Nickel-chelating headgroups ("Ni Lipos"; Fig 3H). In this extraction assay, TA proteins that are extracted from liposomes by ATAD1/Msp1 are bound by soluble GST-tagged chaperones, purified on a glutathione column, and detected by Western blot (39). His-ATAD1 directly and efficiently extracted 3xFLAG-BIML from liposomes in this in vitro assay (Fig 3I). As expected, this activity was ATP-dependent (lanes 17-20) and was abolished when the catalytically inactive mutant, ATAD1E193Q, was used (lanes 1-4). Omitting Ni-chelating lipids ("Mito Lipos;" lanes 5-8) blocked extraction of BIM, demonstrating that ATAD1 requires membrane anchoring for its extractase activity. Importantly, ATAD1 did not extract Fis1, another TA protein (lanes 9-12) . Thus, ATAD1 specifically extracts BIM, but not all TA proteins. (38).

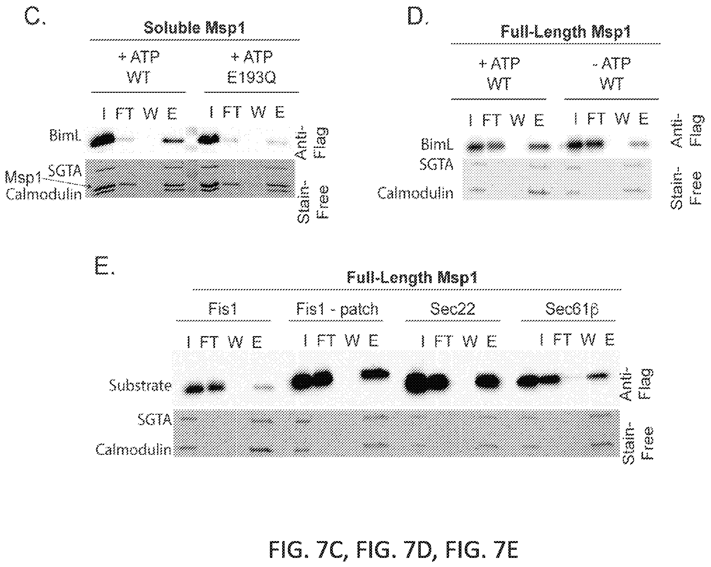

The Ni-His anchoring strategy was validated by using either the full-length (FL) or a similarly His-tagged version of the yeast homolog Msp1 (Fig 7B). Both FL-Msp1 and His-Msp1 extracted BIM (Fig 7C,D), in line with the biochemical conservation of ATAD1 and Msp1. The substrate selectivity of this assay was validated using Msp1 and a series of positive and negative control TA proteins (Fig 7E). In all, these experiments show that ATAD1 directly and specifically extracts BIM from lipid membranes.

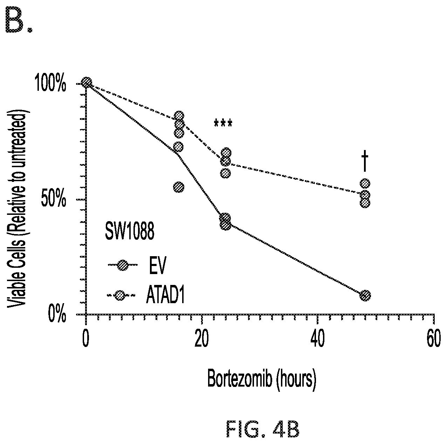

BIM accumulates upon dysfunction of the ubiquitin proteasome system (UPS), induced by stressors such as loss of MARCH5 or treatment with proteasome inhibitors (41). Inducing UPS dysfunction with proteasome inhibitors in Del(10q23) cells can increase BIM and exploit the absence of ATAD1. Indeed, bortezomib and carfilzomib potently and rapidly induced a loss of viability in Del10q23 cells (SW1088 and H4 glioma and RPMI7951 melanoma), which was mitigated by expression of ATAD1 (Fig 4 A,B; Fig 8A-E). There are no available Del(10q23) prostate cancer cell lines, but PC3 cells are PTEN-null and ATAD1-hemizygous (Fig 8F). Deletion of the remaining allele of ATAD1 in PC3 cells sensitized to bortezomib, while overexpression of ATAD1 - but not ATAD1E193Q - increased resistance to bortezomib (Fig 8G-I). Thus, ATAD1 is critically important for cell viability in the context of UPS dysfunction.

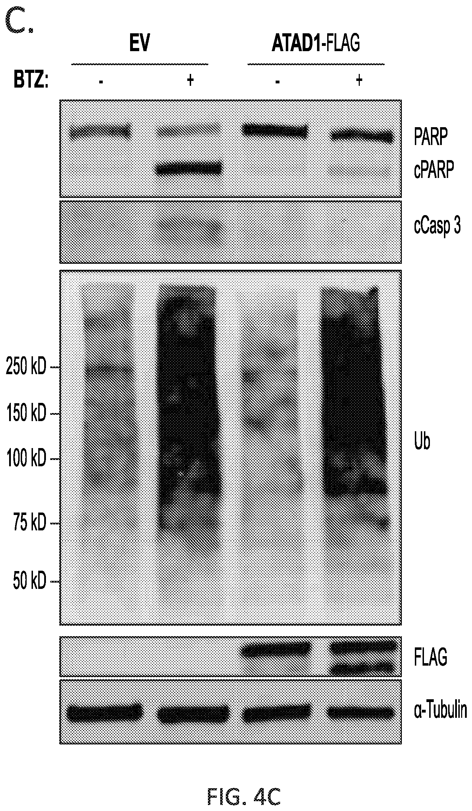

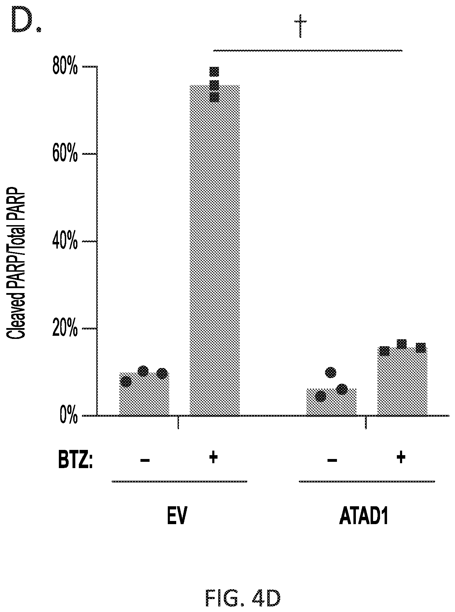

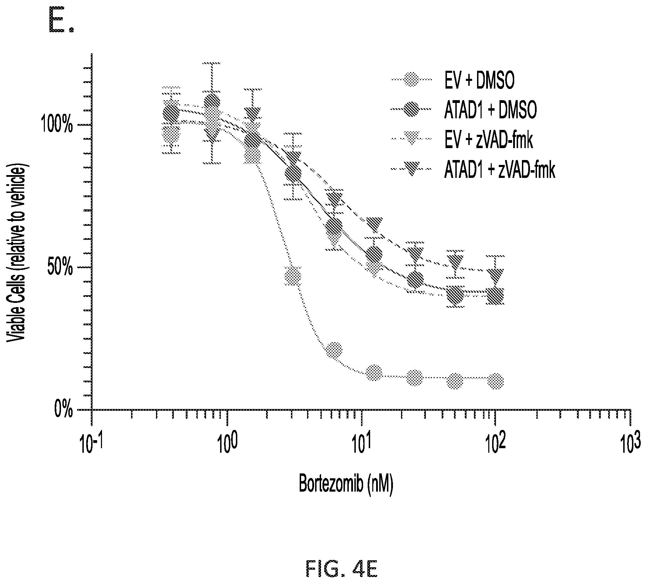

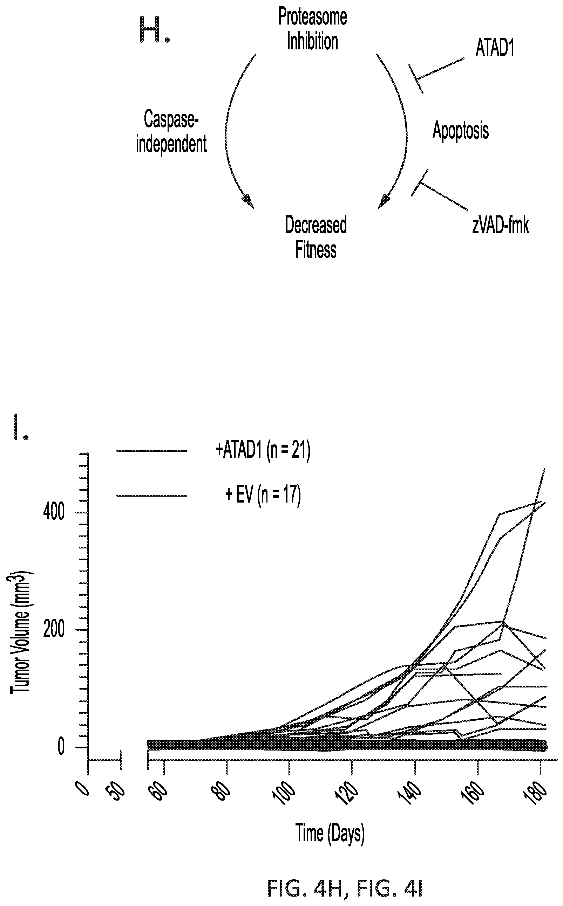

Apoptotic cell death is only one of many mechanisms underlying proteasome inhibitor toxicity in cells (42-44). In our system, bortezomib induced robust apoptosis in Del10q23-EV cells, but only minimally in ATAD1 re-expressing cells (Fig 4C,D). Polyubiquitinated proteins accumulated to the same extent in the presence or absence of ATAD1, indicating that ATAD1 affects how the cell responds to proteotoxic stress, rather than minimizing the proteotoxic insult itself. It was then asked whether ATAD1 affects proteasome inhibitor sensitivity via some apoptosis-independent pathway, in which case ATAD1 re-expression and caspase inhibition (which blocks apoptosis) would have an additive effect in mitigating proteasome inhibitor toxicity. Strikingly, ATAD1 re-expression essentially phenocopied treatment with the caspase inhibitor zVAD-fmk in Del10q23 cells treated with bortezomib (Fig 4E-G). Moreover, there was minimal additive effect of zVAD in ATAD1 re-expressing cells, suggesting that ATAD1 and zVAD act in the same pathway downstream of proteasome inhibition (Fig 4H). Together, these results indicate that the protective effects of ATAD1 during proteasome inhibition can be explained exclusively by apoptosis.

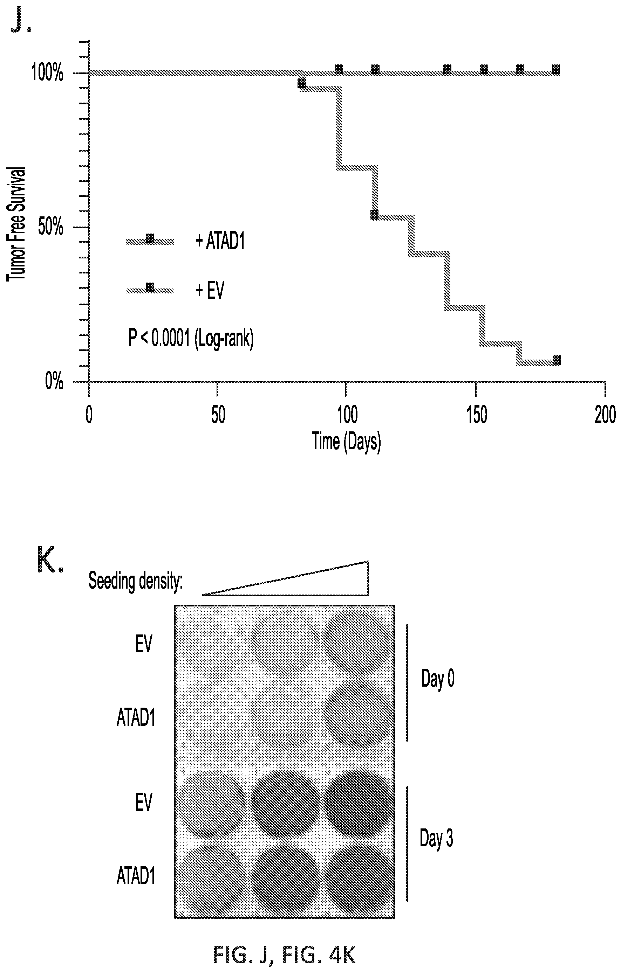

The effect of ATAD1 on tumor growth was assessed in vivo using a subcutaneous xenograft model. SW1088 is a Del10q23 glioma cell line that has low clonogenic potential and is non-tumorigenic in SCID mice (45,46). Accordingly, SW1088 cells transduced with EV failed to form tumors in 17 out of 17 NOD/SCID mice injected. Remarkably, ATAD1 re-expressing cells grew palpable tumors in 17 out of 18 mice injected (Fig 4I,J). This effect cannot be explained simply by proliferation differences, since ATAD1 does not affect the proliferation rate of SW1088 cells in culture (Fig 4K). Thus, ATAD1 serves a profound pro-survival role in PTEN-null cells in vivo, consistent with the findings that ATAD1 extracts BIM for degradation and protects against proteotoxic stress-induced apoptosis.

The delicate balance of pro and anti-apoptotic BCL2 proteins constantly threatens cell survival. How these proteins are activated has been intensely studied, but less is known about how they are degraded. ATAD1 directly and specifically extracts OMM-localized BIM, which accumulates during UPS dysfunction. The finding that ATAD1 loss sensitizes to UPS dysfunction could have significant clinical implications for thousands of cancer patients with Del10q23 tumors.

It might appear counterintuitive that ATAD1 loss occurs so frequently in cancer despite priming cells for apoptosis. One must remember that any time ATAD1 is deleted, PTEN is also deleted. PTEN loss activates AKT, which has strong pro-survival effects, including decreasing transcription of BCL2L11 (BIM) by inhibiting FOXO3A, and inactivating BAD by direct phosphorylation (47,48). Thus, co-deletion of PTEN may buffer some of the apoptotic priming induced by ATAD1 loss. Additionally, genetic lesions that prime cells for apoptosis are not always selected against in cancer. On the contrary, potent oncogenes (including MYC paralogs) can induce apoptotic priming and yet are strongly selected for (49,50). It cannot be ruled out that there is a possibility that ATAD1 loss could be beneficial under some circumstances, but our data overwhelmingly support its role as a pro-survival factor.

Moreover, the effect of ATAD1 on apoptosis likely extends beyond UPS dysfunction and into a broader physiological context. ATAD1 ("Thorase" in mice) has not been reported to be linked to apoptosis, but was originally discovered in mammals via a genetic screen for factors (which included the anti-apoptotic Bcl-xL) that prevent neuronal cell death in response to oxygen and glucose deprivation (OGD) (51). A preconditioning regimen increased the expression of Atad1, which then protected cells from subsequent OGD. Increased expression of ATAD1 in response to pro-survival signals can help cells neutralize pro-apoptotic BIM in other contexts as well. Further, Atad1 phenocopies Bcl2 and Bcl-xL in the middle cerebral artery occlusion mouse model of ischemic stroke: deletion of Atad1 or Bcl2 exacerbates, and overexpression of Atad1, Bcl2, or Bcl2l1 (encoding Bcl-xL) decreases neuronal cell death (52-55). Additionally, it was recently discovered that nitrosylation of a conserved cysteine residue inactivates ATAD1 (56), which could provide a mechanism whereby reactive nitrogen species in injured tissues inactivate ATAD1 to trigger BIM-mediated apoptosis. Thus, antagonism of BIM can contribute to the neuroprotective function of ATAD1/Thorase in vivo, in addition to its established function as a regulator of AMPA receptors. Defining the mechanisms and principles of ATAD1 regulation and function will reshape our understanding of apoptotic susceptibility but also has the potential for profound clinical impact in PTEN/ATAD1 co-deleted tumors and beyond.

### Materials and Methods:

### Lead Contact

Further information and requests for resources and reagents should be directed to and will be fulfilled by the lead contact, Jared Rutter (rutter@biochem.utah.edu).

### Materials Availability

All unique/stable reagents generated within this study are available from the lead contact upon request without restriction.

### Data and Code Availability

The CRISPR screening data generated during this study are available in the supplemental materials.

### EXPERIMENTAL MODEL AND SUBJECT DETAILS

### ATAD1 knockout cell lines

Jurkat E6.1 human T-ALL cells (ATCC TIB-152) were grown in RPMI1640 with 10% FBS and 100 U/mL Pen/Strep (ThermoFisher). Cells were electroporated with using Lonza SE Cell Line 4D-NucleofectorTM X Kit L according to manufacturer's specifications and using the protocol optimized for Jurkat E6.1 cells. Px458-derived plasmids encoding sgRNA targeting ATAD1 were transiently expressed in Jurkat cells via electroporation. Three days later, GFP+ cells were sorted (BD FACSAria) and plated as single cells in 96 well plates. Clonal cell lines were grown, harvested, and evaluated for ATAD1 deletion via immunoblot using a knockout-validated monoclonal antibody (NeuroMab).

PC3 cells were transduced with lentivirus encoding LentiCRISPRv2-GFP (LCv2G) or LCv2G with sgATAD1 (guide sequence #1). Three days after transduction, GFP+ cells were sorted (BD FACSAria) and maintained as a polyclonal population. Editing was confirmed by immunoblot as above.

### ATAD1 Re-expression in Del10q23 cell lines

H4 and PC3 cells were transduced with retrovirus (PQCXIP transfer plasmid) encoding ATAD1 with C-terminal FLAG and HA tags. Two days after transduction, cells were selected with 1 µg/mL puromycin for 4 days. RPMI7951 and SW1088 cells were recalcitrant to retroviral infection, and were instead transduced with lentivirus (pLenti-Blast transfer plasmid) encoding ATAD1-FLAG, and selected with 8 µg/mL blasticidin for 6 days. Cells were grown in media containing the selective antibiotic (puromycin or blasticidin) upon thawing, but no experiments were conducted using media that contained selective antibiotics.

### 1. METHOD DETAILS

### i. Immunohistochemistry

### ATAD1 (using NeuroMab #75-157 mouse monoclonal antibody)

The ATAD1 immunohistochemical staining was performed on 4-micron thick sections of formalin-fixed, paraffin-embedded tissues. Sections were air-dried and then melted in a 60°C oven for 30 minutes. Slides were loaded onto the Leica Bond^{™} III automated staining instrument (Leica Biosystems, Buffalo Grove, IL.) and de-paraffinized with the Bond^{™} Dewax solution. The antigen retrieval performed was done with Bond^{™} Epitope Retrieval Buffer 2 (ER2, pH 8.0) for 20 minutes at 95°C. The ATAD1 primary antibody concentration of 1:400 was applied at an incubation time of 30 minutes at room temperature. Positive signal was visualized using the Bond^{™} Polymer Refine Detection kit-DAB, which is a goat antimouse/anti-rabbit secondary HRP/polymer detection system, utilizing DAB (3-3' diaminobenzidine) as the chromogen. Tissue sections were counterstained with hematoxylin for 10 minutes. The slides were removed from the immunostainer and placed in a dH2O/DAWN ^{™} mixture. The sections were gently washed in a mixture of de-ionized water and DAWN^{™} solution to remove any unbound reagent. The slides were gently rinsed in deionized water until all of wash mixture was removed. The slides were de-hydrated in graded ethanols, cleared in xylene and then coverslipped.

PTEN (using rabbit anti-human monoclonal antibody): clone 138G6, catalog # 9559L, Cell Signaling, Danvers, MA).

The PTEN immunohistochemical staining was performed on 4-micron thick sections of formalin-fixed, paraffin-embedded tissues. Sections were air-dried and then melted in a 60°C oven for 30 minutes. Slides were loaded onto the Ventana BenchMark^{™} Ultra automated staining instrument (Ventana Medical Systems, Tucson, AZ), de-paraffinized with the EZ Prep solution. The antigen retrieval performed was done with a citrate buffer (pH 6.0) in a pressure cooker (BioCare Medical, Concord, CA) for 4 minutes at 100°C then cooled in hot buffer for 30 minutes. The PTEN primary antibody concentration of 1:50 was applied at an incubation time of 2 hours at room temperature. The Ventana Amplification kit was applied to increase the antibody signal. Positive signal was visualized using the UltraView DAB detection kit, which is a goat anti-mouse/anti-rabbit secondary HRP/polymer detection system, utilizing DAB (3-3' diaminobenzidine) as the chromogen. Tissue sections were counterstained with hematoxylin for 16 minutes. The slides were removed from the immunostainer and placed in a dH2O/DAWN ^{™} mixture. The sections were gently washed in a mixture of de-ionized water and DAWN^{™} solution to remove any unbound reagent and coverslip oil applied by the automated instrument. The slides were gently rinsed in deionized water until all of wash mixture was removed. The slides were de-hydrated in graded ethanols, cleared in xylene and then coverslipped.

### ii. Cell Culture

Jurkat cells were cultured in RPMI1640 with 10% FBS (Sigma) and 100 U/mL Pen/Strep. Cells were counted regularly and typically split at a concentration of approximately 1.5 x 106 cells/mL, but always before reaching a concentration of 3x106 cells/mL. Adherent cell lines were maintained in subconfluent cultures in the following media: RPMI1640 (PC3), DMEM (H4, SW1088), EMEM (RPMI7951), all with 10% FBS and 100 µ/mL Pen/Strep.

### iii. Cloning

All cloning was conducted via traditional PCR/restriction enzyme "cut and paste" methods and verified by Sanger sequencing.

### ATAD1 constructs:

Retroviral plasmids encoding ATAD1-FLAG/HA and ATAD1E193Q-FLAG/HA were published previously (Chen et al, 2014). Lentiviral vectors were made, using the pLenti-BLAST backbone, by PCR-amplifying the ATAD1 CDS from the above retroviral vectors, but truncating the construct by replacing the HA tag with a stop codon.

### GFP-BIM Constructs:

The pLVXTet-One vector was purchased from Takara. The coding sequence for EGFP was PCR-amplified and ligated into the MCS using AgeI/BamHI sites. A fusion of EGFP-BIMEL was generated using SOEing PCR and ligated using AgeI/BamHI sites. EGFP-BIMEL was also ligated into pEGFP-C3 for transient transfection.

### iv. Cell Titer Glo Viability Assay

Viability was determined by Cell Titer Glo (Promega) according to the manufacturer's recommendation, with some modifications. Cells were plated at a density of 5 x 103 cells/well in 100 µL in 96 well plates with white walls and clear bottoms (Corning #3610). Cell Titer Glo reagent was reconstituted, diluted 1:4 using sterile PBS, and stored at -20 °C in 10 mL aliquots. For every experiment shown, at least 2 independent experiments were conducted, and each experiment was conducted with 3 biological replicates, except for the ATAD1 overexpression experiment in PC3 cells (in Figure S4I), which had 2 biological replicates each. The outer wells of the 96 well plates were filled with media but not with cells, due to concerns of edge effects. Luminescence was measured using a Biotek Synergy Neo2 microplate reader. Luminescence values were normalized on each plate to untreated cells on the same plate, and expressed as percent.

### v. Crystal Violet Staining

Cells cultured in 12 or 6 well plates were washed twice with PBS then fixed with 4% paraformaldehyde (Sigma Aldrich) for 30 minutes at room temperature. Wells were washed with ddH2O three times, then stained with 0.1% (w/v) crystal violet solution in 20% methanol for 30 minutes at room temperature. Wells were again washed with ddH2O three times, inverted to dry, and plates were photographed against a white background using an iPhone X. For quantification, glacial acetic acid was added to each well to elute the dye, and plates were incubated at room temperature on a rotary shaker for 30 minutes. Absorbance was measured at 590 nm using a Biotek Synergy Neo2 microplate reader, and values were normalized to those from untreated cells of the same genotype on each plate.

### vi. SDS-PAGE and Immunoblotting

Whole cell lysates were prepared by scraping cells directly into RIPA buffer supplemented with protease and phosphatase inhibitors (Sigma Aldrich P8340, Roche Molecular 04906845001), incubated on ice for 30 minutes with vortexing every 10 min, and then spun at 16,000 g for 10 minutes at 4°C to remove insoluble material. Supernatant was saved as lysate. WCL, PMS, and/or crude mitochondrial fractions were normalized for total protein content via BCA Assay (Thermo Scientific 23225). Samples were resolved by SDS-PAGE or Tris-glycine gels (Invitrogen XP04205BOX) and transferred to nitrocellulose or PVDF (extraction assay) membranes. Immunoblotting was performed using the indicated primary antibodies which are listed in the key resources table according to the manufacturers' recommendations, and analyzed by Licor Odyssey or Azure C500 (extraction assay). Note that the detector for the Azure C500 has several columns of pixels which appear to be non-functional. This gives the appearance of thin vertical white lines in some images. This can be readily viewed in raw data files by over-adjusting the contrast.

### vii. Co-Immunoprecipitation

H4 cells (expressing EV or ATAD1-WT-FLAG/HA) were transfected with pEGFP-C3 (GFP) or GFP-BIMEL in pEGFP-C3 (GFP-BIM), 10 µg plasmid for 10 cm plate, in the presence of 20 µM zVAD-fmk. Transient expression proceeded overnight (14-16 hr). Cells were washed with cold PBS and lysed with HN buffer supplemented with protease inhibitor cocktail and 1% CHAPS (HNC buffer). Magnetic anti-FLAG beads (Sigma Aldrich) were equilibrated with HNC buffer and then mixed with lysate (after removing 10% volume as input). Bead-lysate mixtures were incubated on a rotator at 4 °C for 2-4 hr. Beads were washed 3x with HNC buffer, then heated at 65 °C in 30 µL 1X Laemmli buffer for 10 min.

### viii. Cell counting

Cells were counted using BioRad TC20 cell counter. At least two samples were taken from a culture any time a count was to be made and the mean was recorded.

### ix. CRISPR Screen

Jurkat cells (wildtype parental, ATAD1KO #1, and ATAD1KO #2) were transduced by spinfection with a genome-wide lentiviral sgRNA library (Addgene #1000000100; Wang et al, Science 2015) that also encoded Cas9 and a puromycin resistance cassette . Transduction was optimized to achieve an approximate transduction efficiency of 30%, and cells were selected with puromycin (0.5 µg/mL) for three days, allowed to recover without puromycin for two days, then maintained in a lower dose of puromycin (0.2 µg/mL) for the duration of the screen. An initial sample of cells (8 x 107) were collected and frozen at the endpoint of puromycin selection (six days post-transduction). Cells were then maintained in culture for 14 cumulative population doublings. Cells were passaged every two days and seeded into new flasks at a density of 2 x 105 cells/mL. After 14 population doublings, representative samples were collected (8 x 107 cells). As described elsewhere (Adelmann et al, Methods in Mol Biol, 2019), cell pellets were processed using a QIAamp DNA Blood Maxiprep, sgRNA sequences were amplified by PCR, and amplicons were sequenced for 40 cycles by Illumina HiSeq NGS at the Whitehead Institute DNA Sequencing Core Facility.

Sequencing reads were aligned to the sgRNA library, given a pseudocount of 1, and the abundance of each sgRNA was calculated as described previously (Wang et al, Science 2015; Kanarek et al, Nature 2018). The counts from each sample were normalized for sequencing depth. sgRNAs with fewer than 50 reads, and genes with fewer than 4 sgRNAs, in the initial reference dataset were omitted from downstream analyses. The log2 fold change in abundance of each sgRNA between the final and initial reference populations was calculated and used to define a CRISPR score for each gene. The CRISPR score is the average log2 fold change in abundance of all sgRNAs targeting a given gene. The distribution of all sgRNAs targeting a given gene was tested against the entire sgRNA distribution using the Kolmogorov-Smirnov test, and p-values were adjusted using the Benjamini-Hochberg procedure. To achieve a direct comparison of gene essentiality in an ATAD1Δ clone to that in the WT control, sgRNAs that were not adequately represented were omitted (i.e. < 50 reads at the initial time point) in both groups. This step enables a paired analysis of sgRNA changes in abundance, and avoids including a given sgRNA that "scored" in one genetic background but whose effects cannot be assessed in another. Differential CRISPR Scores (dCS) for each ATAD1Δ clone were calculated for each gene as: CSATAD1Δ - CSATAD1-WT. The mean of the two dCS values were summed and are presented as "dCS Combined." Genes that scored as "hits" in one clone but not the other could reflect a clonal artifact or simple experimental error - These genes were penalized in the analysis by combining the Q values (Benjamini-Hochberg-corrected P values) for the two clones via Fisher's Method (Fisher, R. A., 1934; also known as "sum of logs" or "chi-square" method) for meta-analysis using the R package "metap" (Dewey M, 2020). Data were analyzed and plotted using R version 4 and RStudio version 1.1.442

### x. BH3-Profiling

BH3-profiling was conducted using a FACS based method to directly monitor Cytochrome C release/retention in cells, as described previously (Ryan and Letai, Methods 2013).

### xi. Fluorescence Microscopy

SW1088 cells (expressing EV or ATAD1-WT) were grown on 8-chamber slides, seeded at 5 x 103 cells per chamber 2 days before imaging. The night before imaging (12 hr), cells were treated with 10 nM bortezomib (BTZ) or untreated. The next morning, chambers were washed twice with PBS, and stained with MitoTracker Red CMXRos (20 nM) for 20 min in the 37 °C CO2 incubator. Cells were washed twice with media then immediately imaged on an Axio Observer Z1 imaging system (Carl Zeiss) equipped with 40× and 100× objectives (oil-immersion). At least 30 cells were imaged for each condition and repeated for n = 2 independent experiments.

### xii. Mouse Xenografts

SW1088 cells (transduced with EV or ATAD1-FLAG) were grown under normal culture conditions, as described above. Cells (3 x 106) were mixed 1: 1 with Matrigel (Corning^{®}) and injected into one flank per mouse. Mice were male NOD/SCID aged 13-15 weeks. Tumor volumes were monitored biweekly using a Biopticon TumorImager. Animal experiments were in concordance with The University of Utah IACUC.

### xiii. Bacterial transformation

For cloning, E. coli DH5□ competent cells (New England Biolabs) were transformed according to the manual provided by the manufacturer and grown on LB agar plates at 37□ C overnight. For cloning of lentiviral and retroviral vectors, NEB Stable competent cells were used (NEB C3040I).

### xiv. E. cloni cells

For cloning, E cloni10G competent cells were transformed according to the manual provided by the manufacturer (Lucigen) and grown on LB agar plates at 37□ C overnight.

### xv. BL21-DE3 pRIL cells

For protein expression, E. coli BL21(DE3) containing a pRIL plasmid and a protein expression vector were grown in terrific broth at 37□ C until an OD600 of 0.6-1.0. Cultures were induced with isopropyl-1-thio-□-D-galactopyranoside (IPTG) at a final concentration of 1 mM and grown at room temperature for an additional 3-4 h.

### xvi. Production of soluble constructs

### Δ1-32 Msp1 and Δ 1-39ATAD1:

The gene encoding the soluble region of S. cerevisiae Msp1 (Δ 1-32) was PCR amplified from genomic DNA and subcloned into a pET28a derivative (Novagen) encoding an N-terminal 6xHis tag followed by a TEV protease cleavage site. The soluble region of Rattus norvegicus ATAD1 (Δ 1-39) was PCR amplified from a plasmid containing ATAD1 cDNA (GE Healthcare). All insertions and deletions were performed by standard PCR techniques. Site-specific mutagenesis was carried out by QuickChange PCR. All constructs were verified by DNA sequencing.

Plasmids encoding soluble Msp1, ATAD1, or their mutants were purified as described previously (Wohlever et al., 2017). Plasmids were transformed into E. coli BL21(DE3) containing a pRIL plasmid and expressed in terrific broth at 37° C until an OD600 of 0.6-1.0, cultures were induced with 1 mM IPTG and grown at room temperature for an additional 3-4 h. Cells were harvested by centrifugation, and resuspended in Msp1 Lysis Buffer (20 mM Tris pH 7.5, 200 mM KAc, 20 mM Imidazole, 0.01 mM EDTA, 1 mM DTT) supplemented with 0.05 mg/mL lysozyme (Sigma), 1 mM phenylmethanesulfonyl fluoride (PMSF) and 500 U of universal nuclease (Pierce), and lysed by sonication. The supernatant was isolated by centrifugation for 30 min at 4° C at 18,500 x g and purified by Ni-NTA affinity chromatography (Pierce) on a gravity column. Ni-NTA resin was washed with 10 column volumes (CV) of Msp1 Lysis Buffer and then 10 CV of Wash Buffer (Msp1 Lysis buffer with 30 mM Imidazole) before elution with Lysis Buffer supplemented with 250 mM imidazole. Purification of soluble ATAD1 also included the addition of ATP to stabilize the protein. ATP was added to a final concentration of 2 mM after sonication and again after elution from the nickel resin.

The protein was further purified by size exclusion chromatography (SEC) (Superdex 200 Increase 10/300 GL, GE Healthcare) in 20 mM Tris pH 7.5, 200 mM KAc, 1 mM DTT. Peak fractions were pooled, concentrated to 5-15 mg/ml in a 30 kDa MWCO Amicon Ultra centrifugal filter (Pierce) and aliquots were flash-frozen in liquid nitrogen and stored at -80 °C. Protein concentrations were determined by A280 using a calculated extinction coefficient (Expasy).

### xvii. GST-SGTA and GST-calmodulin:

GST tagged SGTA was expressed and purified as described previously(Mateja et al., 2015). The original calmodulin plasmid was a kind gift of the Hegde lab (Shao and Hegde, 2011). Calmodulin was cloned into pGEX6p1 plasmid by standard methods. GST-SGTA and GST-calmodulin were expressed as described above for soluble Msp1 constructs. Cells were harvested by centrifugation and resuspended in SGTA Lysis Buffer (50 mM Hepes pH 7.5, 150 mM NaCl, 0.01 mM EDTA, 1 mM DTT, 10% glycerol) supplemented with 0.05 mg/mL lysozyme (Sigma), 1 mM PMSF and 500 U of universal nuclease (Pierce), and lysed by sonication. The supernatant was isolated by centrifugation for 30 min at 4□ C at 18,500 x g and purified by Glutathione affinity chromatography (Thermo Fisher) on a gravity column. Resin was washed with 20 column volumes (CV) of SGTA Lysis Buffer and then eluted with 3 CV of SGTA Lysis Buffer supplemented with 10 mM reduced glutathione. The protein was further purified by size exclusion chromatography (SEC) (Superdex 200 Increase 10/300 GL, GE Healthcare) in 20 mM Tris pH 7.5, 100 mM NaCl, 0.1 mM TCEP. Peak fractions were pooled, concentrated to 10 mg/ml in a 30 kDa MWCO Spin Concentrator (Pierce) and aliquots were flash-frozen in liquid nitrogen and stored at -80 °C. Protein concentrations were determined by A280 using a calculated extinction coefficient (Expasy).

### xviii. Production of membrane proteins

### BIM and Fis1:

Homo sapiens BimL or S. cerevisiae Fis1 TMD +/- 5 flanking amino acids (residues 126-155) was cloned in place of the Sec22 TMD in the SumoTMD construct described previously (Wang et al., 2010; Wohlever et al., 2017). These constructs have N-terminal His6 and 3x Flag tags and a C-terminal opsin glycosylation site (11 residues). A 3C protease site was added immediately after the His tag by standard PCR methods. The resulting constructs are His6-3C-3xFlag-Sumo-thrombin-BimL-Opsin and His6-3C-3xFlag-Sumo-thrombin-Fis1(126-155)-Opsin.

Expression plasmids for SumoTMD were transformed into E. coli BL21(DE3) containing a pRIL plasmid and expressed in terrific broth at 37° C until an OD600 of 0.6-0.8, cultures were induced with 0.4 mM IPTG and grown at 20° C for an additional 3-4 h. Cells were harvested by centrifugation, and resuspended in SumoTMD Lysis Buffer (50 mM Tris pH 7.5, 300 mM NaCl, 10 mM MgCl2, 10 mM Imidazole, 10% glycerol) supplemented with 0.05 mg/mL lysozyme (Sigma), 1 mM PMSF and 500 U of universal nuclease (Pierce), and lysed by sonication. Membrane proteins were solubilized by addition of n-dodecyl-□-D-maltoside (DDM) to a final concentration of 1% and rocked at 4° C for 30'. Lysate was cleared by centrifugation for at 4° C for 1 h at 35,000 x g and purified by Ni-NTA affinity chromatography.

Ni-NTA resin was washed with 10 column volumes (CV) of SumoTMD Wash Buffer 1 (50 mM Tris pH 7.5, 500 mM NaCl, 10 mM MgCl2, 10 mM imidazole, 5 mM β-mercaptoethanol (BME), 10% glycerol, 0.1% DDM). Resin was then washed with 10 CV of SumoTMD Wash Buffer 2 (same as Wash Buffer 1 except with 300 mM NaCl and 25 mM imidazole) and 10 CV of SumoTMD Wash Buffer 3 (same as Wash Buffer 1 with 150 mM NaCl and 50 mM imidazole) and then eluted with 3 CV of SumoTMD Elution Buffer (same as Wash Buffer 3 except with 250 mM imidazole).

The protein was further purified by size exclusion chromatography (SEC) (Superdex 200 Increase 10/300 GL, GE Healthcare) in 50 mM Tris pH 7.5, 150 mM NaCl, 10 mM MgCl2, 5 mM BME, 10% glycerol, 0.1% DDM. Peak fractions were pooled and concentrated in a 30 kDa MWCO spin concentrator (Pierce). Sample was then incubated with 3C Protease at a 1:100 ratio at 4□ C overnight to remove the His tag. The following day, the sample was run over Ni-NTA resin equilibrated in Lysis Buffer to remove 3C protease, His tag, and uncleaved proteins. Flow through was collected, aliquoted, and flash-frozen in liquid nitrogen and stored at -80 °C. Protein concentrations were determined by A280 using a calculated extinction coefficient (Expasy).

### Msp1:

Full-length S. cerevisiae Msp1 was PCR amplified from genomic DNA, subcloned into a pET21b derivative with a C-terminal 6xHis tag and expressed as described above for the soluble constructs. Cells were lysed by sonication and the insoluble fraction was harvested by centrifugation for 1 h at 4□ C at 140,000 x g. After resolubilizing for 16 h in Msp1 Lysis Buffer containing 1% DDM (Bioworld), the detergent-soluble supernatant was isolated by centrifugation for 45 min at 142,000 x g and purified by Ni-NTA affinity chromatrography and SEC as described above for the soluble constructs, except that all buffers contained 0.05% DDM. Peak fractions were concentrated in 100 kDa MWCO Amicon Ultra centrifugal filter (Millipore). Protein concentrations were determined by A280 using a calculated extinction coefficient (Expasy) and aliquots were flash frozen in liquid nitrogen.

### xix. Reconstitution of Msp1 activity in proteoliposomes

### Liposome preparation

Liposomes mimicking the lipid composition of the yeast outer mitochondrial membrane were prepared as described (Kale et al., 2014). Briefly, a 25 mg lipid film was prepared by mixing chloroform stocks of chicken egg phosphatidyl choline (Avanti 840051C), chicken egg phosphatidyl ethanolamine (Avanti 840021C), bovine liver phosphatidyl inositol (Avanti 840042C), synthetic DOPS (Avanti 840035C), and synthetic TOCL (Avanti 710335C) at a 48:28:10:10:4 molar ratio with 1 mg of DTT. Nickel liposomes were made as described above, except, 1,2-dioleoyl-sn-glycero-3-[N-(5-amino-1-carboxypentyl)iminodiacetic acid)succinyl] Nickel salt (Avanti 790404) was used at a molar ratio of 2% and DOPS was dropped from 10% to 8%.

Chloroform was evaporated under a gentle steam of nitrogen and then left on a vacuum (<1 mTorr) overnight. Lipid film was resuspended in Liposome Buffer (50 mM Hepes KOH pH 7.5, 15% glycerol, 1 mM DTT) to a final concentration of 20 mg/mL and then subjected to five freeze-thaw cycles with liquid nitrogen. Liposomes were extruded 15 times through a 200 nm filter at 60° C, distributed into single use aliquots, and flash frozen in liquid nitrogen.

### Proteoliposome preparation

For extraction assays with full-length Msp1, proteoliposomes were prepared by mixing 1 µM Msp1, 1 µM TA protein (SumoTMD), and 2 mg/mL of mitochondrial liposomes in Reconstitution Buffer (50 mM Hepes KOH pH 7.5, 200 mM potassium acetate, 7 mM magnesium acetate, 2 mM DTT, 10% sucrose, 0.01% sodium azide, and 0.1% deoxy big chaps)(Zhang et al., 2013). For extraction assays with soluble Msp1/ATAD1, proteoliposomes were prepared by mixing 1 µM TA protein (SumoTMD), and 2 mg/mL of Nickel liposomes in Reconstitution Buffer. Detergent was removed by adding 25 mg of biobeads and rotating the samples for 16 h at 4 °C. After removing biobeads, unincorporated TA protein was pre-cleared by incubating the reconstituted material with excess (5 µM) GST-SGTA and GST-Calmodulin and passing over a glutathione spin column (Pierce #16103); the flow through was collected and used immediately for dislocation assays.

### xx. Extraction Assay

Extraction assays contained 60 µL of pre-cleared proteoliposomes, 5 µM GST-SGTA, 5 µM calmodulin, and 2 mM ATP and the final volume was adjusted to 200 µL with Extraction Buffer (50 mM Hepes KOH pH 7.5, 200 mM potassium acetate, 7 mM magnesium acetate, 2 mM DTT, 0.1 µM calcium chloride). Samples were incubated at 30° C for 35 min and then loaded onto a glutathioine spin column. Columns were washed 4x with Extraction Buffer and eluted with the same buffer supplemented with 20 mM glutathione pH 8.5. Samples were loaded onto stain free gels, imaged, and then transferred to a PVDF membrane and blotted as indicated in the key resource table. To account for variability in reconstitution efficiency and western blotting, a new reconstitution and dislocation assay with wild-type Msp1 was done in parallel with each mutant Msp1. Figures are representative of N > 3 separate reconstitutions. Note that the "input" lane is diluted 5x relative to the "elution" lane.

### xxi. Quantification and Statistical Analysis

Western blot band intensities were estimated using ImageJ (Schneider et al., 2012). To account for variability in reconstitution efficiency and western blotting, a new reconstitution and dislocation assay with wild-type Msp1 was done in parallel with each Msp1 mutant. Figures are representative of N > 3 separate reconstitutions. Dislocation efficiency was quantified by comparing the amount TA protein in the "elution" lane with the amount of substrate in the "input" lane.

### References

1. Worby CA, Dixon JE. Pten. Annu Rev Biochem. 2014;83(1):641-69.
2. Chen Y-C, Umanah GKE, Dephoure N, Andrabi SA, Gygi SP, Dawson TM, et al. Msp1/ATAD1 maintains mitochondrial function by facilitating the degradation of mislocalized tail-anchored proteins. EMBO J. 2014 Jul 17;33(14):1548-64.
3. Okreglak V, Walter P. The conserved AAA-ATPase Msp1 confers organelle specificity to tail-anchored proteins. Proc Natl Acad Sci U S A. 2014 Jun 3;111(22):8019-24.
4. Nakai M, Endo T, Hase T, Matsubara H. Intramitochondrial protein sorting. Isolation and characterization of the yeast MSP1 gene which belongs to a novel family of putative ATPases. J Biol Chem. 1993 Nov 15;268(32):24262-9.
5. Wang L, Walter P. Msp1/ATAD1 in Protein Quality Control and Regulation of Synaptic Activities. Annu Rev Cell Dev Biol. 2020;36(1):141-64.
6. Zhang J, Wang Y, Chi Z, Keuss MJ, Pai Y-ME, Kang HC, et al. The AAA+ ATPase Thorase Regulates AMPA Receptor-Dependent Synaptic Plasticity and Behavior. Cell. 2011 Apr 15;145(2):284-99.
7. Ahrens-Nicklas RC, Umanah GKE, Sondheimer N, Deardorff MA, Wilkens AB, Conlin LK, et al. Precision therapy for a new disorder of AMPA receptor recycling due to mutations in ATAD1. Neurol Genet. 2017 Feb 1;3(1):e130.
8. Poluri RTK, Audet-Walsh É. Genomic Deletion at 10q23 in Prostate Cancer: More Than PTEN Loss? Front Oncol [Internet]. 2018 Jun 29 [cited 2020 Apr 19];8. Available from: https://www.ncbi.nlm.nih.gov/pmc/articles/PMC6033966/
9. Chung JH, Dewal N, Sokol E, Mathew P, Whitehead R, Millis SZ, et al. Prospective Comprehensive Genomic Profiling of Primary and Metastatic Prostate Tumors. JCO Precis Oncol [Internet]. 2019 May 10 [cited 2020 May 1]; Available from: https://ascopubs.org/doi/pdf/10.1200/PO.18.00283
10. Weir NR, Kamber RA, Martenson JS, Denic V. The AAA protein Msp1 mediates clearance of excess tail-anchored proteins from the peroxisomal membrane. eLife. 2017 Sep 14;6:e28507.
11. Weidberg H, Amon A. MitoCPR-A surveillance pathway that protects mitochondria in response to protein import stress. Science [Internet]. 2018 Apr 13 [cited 2020 Apr 24];360(6385). Available from: https://science.sciencemag.org/content/360/6385/eaan4146
12. Basch M, Wagner M, Rolland S, Carbonell A, Zeng R, Khosravi S, et al. Msp1 cooperates with the proteasome for extraction of arrested mitochondrial import intermediates. Mol Biol Cell. 2020 Feb 12;31(8):753-67.
13. Muller FL, Aquilanti EA, DePinho RA. Collateral Lethality: A New Therapeutic Strategy in Oncology. Trends Cancer. 2015 Nov 1;1(3):161-73.
14. Muller FL, Colla S, Aquilanti E, Manzo V, Genovese G, Lee J, et al. Passenger Deletions Generate Therapeutic Vulnerabilities in Cancer. Nature. 2012 Aug 16;488(7411):337-42.
15. Dewey, Michael. metap: meta-analysis of significance values. R Package Version 14. 2020;
16. Chen D, Latham J, Zhao H, Bisoffi M, Farelli J, Dunaway-Mariano D. Human Brown Fat Inducible Thioesterase Variant 2 Cellular Localization and Catalytic Function. Biochemistry. 2012 Sep 4;51(35):6990-9.
17. Bekeova C, Anderson-Pullinger L, Boye K, Boos F, Sharpadskaya Y, Herrmann JM, et al. Multiple mitochondrial thioesterases have distinct tissue and substrate specificity and CoA regulation, suggesting unique functional roles. J Biol Chem. 2019 Dec 13;294(50):19034-47.
18. Nakamura N, Kimura Y, Tokuda M, Honda S, Hirose S. MARCH-V is a novel mitofusin 2- and Drp1-binding protein able to change mitochondrial morphology. EMBO Rep. 2006 Aug 1;7(10):1019-22.
19. Cherok E, Xu S, Li S, Das S, Meltzer WA, Zalzman M, et al. Novel regulatory roles of Mff and Drp1 in E3 ubiquitin ligase MARCH5-dependent degradation of MiD49 and Mcl1 and control of mitochondrial dynamics. Mol Biol Cell. 2016 Dec 8;28(3):396-410.
20. Yonashiro R, Ishido S, Kyo S, Fukuda T, Goto E, Matsuki Y, et al. A novel mitochondrial ubiquitin ligase plays a critical role in mitochondrial dynamics. EMBO J. 2006 Aug 9;25(15):3618-26.
21. Kale J, Osterlund EJ, Andrews DW. BCL-2 family proteins: changing partners in the dance towards death. Cell Death Differ. 2018 Jan;25(1):65-80.
22. Letai A. Apoptosis and Cancer. Annu Rev Cancer Biol. 2017;1(1):275-94.
23. Czabotar PE, Lessene G, Strasser A, Adams JM. Control of apoptosis by the BCL-2 protein family: implications for physiology and therapy. Nat Rev Mol Cell Biol. 2014 Jan;15(1):49-63.
24. Llambi F, Moldoveanu T, Tait SWG, Bouchier-Hayes L, Temirov J, McCormick LL, et al. A Unified Model of Mammalian BCL-2 Protein Family Interactions at the Mitochondria. Mol Cell. 2011 Nov 18;44(4):517-31.
25. Greaves G, Milani M, Butterworth M, Carter RJ, Byrne DP, Eyers PA, et al. BH3-only proteins are dispensable for apoptosis induced by pharmacological inhibition of both MCL-1 and BCL-X L. Cell Death Differ. 2018 Sep 5;1.
26. Djajawi TM, Liu L, Gong J, Huang AS, Luo M, Xu Z, et al. MARCH5 requires MTCH2 to coordinate proteasomal turnover of the MCL1:NOXA complex. Cell Death Differ. 2020 Feb 24;1-16.
27. Haschka MD, Karbon G, Soratroi C, O'Neill KL, Luo X, Villunger A. MARCH5-dependent degradation of MCL1/NOXA complexes defines susceptibility to antimitotic drug treatment. Cell Death Differ [Internet]. 2020 Feb 3 [cited 2020 Apr 19]; Available from: http://www.nature.com/articles/s41418-020-0503-6
28. Subramanian A, Andronache A, Li Y-C, Wade M. Inhibition of MARCH5 ubiquitin ligase abrogates MCL1-dependent resistance to BH3 mimetics via NOXA. Oncotarget. 2016 Feb 21;7(13):15986-6002.
29. Arai S, Varkaris A, Nouri M, Chen S, Xie L, Balk SP. MARCH5 mediates NOXA-dependent MCL1 degradation driven by kinase inhibitors and integrated stress response activation. Macleod KF, Murphy ME, editors. eLife. 2020 Jun 2;9:e54954.
30. DeWeirdt PC, Sangree AK, Hanna RE, Sanson KR, Hegde M, Strand C, et al. Genetic screens in isogenic mammalian cell lines without single cell cloning. Nat Commun. 2020 Feb 6;11(1):1-15.
31. Wang T, Yu H, Hughes NW, Liu B, Kendirli A, Klein K, et al. Gene Essentiality Profiling Reveals Gene Networks and Synthetic Lethal Interactions with Oncogenic Ras. Cell. 2017 Feb 23;168(5):890-903.e15.
32. Caenepeel S, Brown SP, Belmontes B, Moody G, Keegan KS, Chui D, et al. AMG 176, a Selective MCL1 Inhibitor, is Effective in Hematological Cancer Models Alone and in Combination with Established Therapies. Cancer Discov. 2018 Sep 25;CD-18-0387.
33. Letai A, Bassik MC, Walensky LD, Sorcinelli MD, Weiler S, Korsmeyer SJ. Distinct BH3 domains either sensitize or activate mitochondrial apoptosis, serving as prototype cancer therapeutics. Cancer Cell. 2002 Sep;2(3):183-92.
34. Ley R, Ewings KE, Hadfield K, Cook SJ. Regulatory phosphorylation of Bim: sorting out the ERK from the JNK. Cell Death Differ. 2005 Aug;12(8):1008-14.
35. Liu Q, Osterlund EJ, Chi X, Pogmore J, Leber B, Andrews DW. Bim escapes displacement by BH3-mimetic anti-cancer drugs by double-bolt locking both Bcl-XL and Bcl-2. Dötsch V, Cole PA, editors. eLife. 2019 Mar 12;8:e37689.
36. Chi X, Nguyen D, Pemberton JM, Osterlund EJ, Liu Q, Brahmbhatt H, et al. The carboxyl-terminal sequence of bim enables bax activation and killing of unprimed cells. Dötsch V, Cole PA, Dötsch V, Borner C, editors. eLife. 2020 Jan 24;9:e44525.
37. Castanzo DT, LaFrance B, Martin A. The AAA+ ATPase Msp1 is a processive protein translocase with robust unfoldase activity. Proc Natl Acad Sci. 2020 Jun 30;117(26):14970-7.
38. Li L, Zheng J, Wu X, Jiang H. Mitochondrial AAA-ATPase Msp1 detects mislocalized tail-anchored proteins through a dual-recognition mechanism. EMBO Rep. 2019 Apr 1;20(4):e46989.
39. Wohlever ML, Mateja A, McGilvray PT, Day KJ, Keenan RJ. Msp1 Is a Membrane Protein Dislocase for Tail-Anchored Proteins. Mol Cell. 2017 Jul 20;67(2):194-202.e6.
40. Hübner A, Barrett T, Flavell RA, Davis RJ. Multisite Phosphorylation Regulates Bim Stability and Apoptotic Activity. Mol Cell. 2008 May 23;30(4):415-25.
41. Puthalakath H, O'Reilly LA, Gunn P, Lee L, Kelly PN, Huntington ND, et al. ER Stress Triggers Apoptosis by Activating BH3-Only Protein Bim. Cell. 2007 Jun 29;129(7):1337-49.
42. Tsvetkov P, Detappe A, Cai K, Keys HR, Brune Z, Ying W, et al. Mitochondrial metabolism promotes adaptation to proteotoxic stress. Nat Chem Biol. 2019 Jul;15(7):681-9.
43. Schneider K, Bertolotti A. Surviving protein quality control catastrophes - from cells to organisms. J Cell Sci. 2015 Nov 1;128(21):3861-9.
44. Huang HH, Ferguson ID, Thornton AM, Bastola P, Lam C, Lin Y-HT, et al. Proteasome inhibitor-induced modulation reveals the spliceosome as a specific therapeutic vulnerability in multiple myeloma. Nat Commun [Internet]. 2020 Dec [cited 2020 Apr 24];11(1). Available from: http://www.nature.com/articles/s41467-020-15521-4
45. Jiang L, Zhou J, Zhong D, Zhou Y, Zhang W, Wu W, et al. Overexpression of SMC4 activates TGFβ/Smad signaling and promotes aggressive phenotype in glioma cells. Oncogenesis. 2017 Mar;6(3):e301-e301.
46. Mercapide J, Cicco RLD, Castresana JS, Klein-Szanto AJP. Stromelysin-1/matrix metalloproteinase-3 (MMP-3) expression accounts for invasive properties of human astrocytoma cell lines. Int J Cancer. 2003;106(5):676-82.
47. Gilley J, Coffer PJ, Ham J. FOXO transcription factors directly activate bim gene expression and promote apoptosis in sympathetic neurons. J Cell Biol. 2003 Aug 18;162(4):613-22.
48. Datta SR, Dudek H, Tao X, Masters S, Fu H, Gotoh Y, et al. Akt Phosphorylation of BAD Couples Survival Signals to the Cell-Intrinsic Death Machinery. Cell. 1997 Oct 17;91(2):231-41.
49. Mason KD, Vandenberg CJ, Scott CL, Wei AH, Cory S, Huang DCS, et al. In vivo efficacy of the Bcl-2 antagonist ABT-737 against aggressive Myc-driven lymphomas. Proc Natl Acad Sci. 2008 Nov 18;105(46):17961-6.
50. Dammert MA, Brägelmann J, Olsen RR, Böhm S, Monhasery N, Whitney CP, et al. MYC paralog-dependent apoptotic priming orchestrates a spectrum of vulnerabilities in small cell lung cancer. Nat Commun [Internet]. 2019 Dec [cited 2019 Aug 4];10(1). Available from: http://www.nature.com/articles/s41467-019-11371-x
51. Dai C, Liang D, Li H, Sasaki M, Dawson TM, Dawson VL. Functional Identification of Neuroprotective Molecules. Deli MA, editor. PLoS ONE. 2010 Nov 24;5(11):e15008.
52. Zhang J, Yang J, Wang H, Sherbini O, Keuss MJ, Umanah GK, et al. The AAA + ATPase Thorase is neuroprotective against ischemic injury. J Cereb Blood Flow Metab. 2019 Sep 1;39(9):1836-48.
53. Martinou J-C, Dubois-Dauphin M, Staple JK, Rodriguez I, Frankowski H, Missotten M, et al. Overexpression of BCL-2 in transgenic mice protects neurons from naturally occurring cell death and experimental ischemia. Neuron. 1994 Oct 1;13(4):1017-30.
54. Parsadanian AS, Cheng Y, Keller-Peck CR, Holtzman DM, Snider WD. Bcl-xL is an Antiapoptotic Regulator for Postnatal CNS Neurons. J Neurosci. 1998 Feb 1;18(3):1009-19.
55. Broughton Brad R.S., Reutens David C., Sobey Christopher G. Apoptotic Mechanisms After Cerebral Ischemia. Stroke. 2009 May 1;40(5):e331-9.
56. Umanah GKE, Ghasemi M, Yin X, Chang M, Kim JW, Zhang J, et al. AMPA Receptor Surface Expression Is Regulated by S-Nitrosylation of Thorase and Transnitrosylation of NSF. Cell Rep [Internet]. 2020 Nov 3 [cited 2020 Dec 13];33(5). Available from: https://www.cell.com/cell-reports/abstract/S2211-1247(20)31318-8

## Claims

1. Proteasome inhibitor for use in treating cancer in a subject, wherein the subject has a non-functional ATAD1 gene, wherein the proteasome inhibitor is bortezomib or carfilzomib.

2. The proteasome inhibitor for use in treating cancer according to claim 1, wherein the subject further comprises a non-functional PTEN gene.

3. The proteasome inhibitor for use in treating cancer of claim 1 or 2, wherein a portion or all of the ATAD1 gene is deleted, and/or wherein a portion or all of the PTEN gene is deleted.

4. The proteasome inhibitor for use in treating cancer of claims 1 to 3, wherein the subject has previously been diagnosed as being susceptible to treatment by a proteasome inhibitor which comprises identifying the subject as having a non-functional ATAD1 gene.

5. The proteasome inhibitor for use in treating cancer of claims 1 to 4, wherein diagnosing the subject as being susceptible to treatment by a proteasome inhibitor further comprises identifying the subject as having a non-functional PTEN gene.

6. The proteasome inhibitor for use in treating cancer of claims 1 to 5, wherein diagnosing the subject as being susceptible to treatment by a proteasome inhibitor further comprises detecting an increase in BIM levels.

7. Proteasome inhibitor for use in treating cancer in a subject by increasing apoptosis in said a subject, wherein the subject comprises a non-functional ATAD1 gene, wherein the proteasome inhibitor is bortezomib or carfilzomib.

8. The proteasome inhibitor for use in treating cancer of claim 7, wherein the subject further comprises a non-functional PTEN gene.

9. The proteasome inhibitor for use in treating cancer of claim 7 or 8, wherein the subject having a non-functional ATAD1 gene is a subject having a portion or all of the ATAD1 gene deleted.

10. The proteasome inhibitor for use in treating cancer of claims 7 to 9, wherein subject having a non-functional PTEN gene is a subject having a portion or all of the PTEN gene deleted.

11. The proteasome inhibitor for use in treating cancer of claims 7 to 10, wherein the subject having cancer has breast cancer, lung cancer, colon cancer, brain cancer, or prostate cancer.

## Patentansprüche

1. Proteasom-Inhibitor zur Verwendung bei der Behandlung von Krebs bei einem Patienten, wobei der Patient ein nicht funktionsfähiges ATAD1-Gen aufweist, wobei der Proteasom-Inhibitor Bortezomib oder Carfilzomib ist.

2. Proteasom-Inhibitor nach Patentanspruch 1, wobei der Patient ferner ein nicht funktionsfähiges PTEN-Gen aufweist.

3. Proteasom-Inhibitor nach Patentanspruch 1 oder 2 zur Verwendung bei der Behandlung von Krebs, wobei ein Teil oder das gesamte ATAD1-Gen deletiert ist und/oder wobei ein Teil oder das gesamte PTEN-Gen deletiert ist.

4. Proteasom-Inhibitor nach den Patentansprüchen 1 bis 3 zur Verwendung bei der Behandlung von Krebs, wobei bei dem Patienten zuvor eine Empfänglichkeit für eine Behandlung mit einem Proteasom-Inhibitor diagnostiziert wurde, wobei die Diagnose umfasst, dass bei dem Patienten ein nicht funktionsfähiges ATAD1-Gen identifiziert wird.

5. Proteasom-Inhibitor nach den Patentansprüchen 1 bis 4 zur Verwendung bei der Behandlung von Krebs, wobei bei dem Patienten zuvor eine Empfänglichkeit für eine Behandlung mit einem Proteasom-Inhibitor diagnostiziert wurde, wobei die Diagnose ferner das Identifizieren des Patienten als Träger eines nicht funktionsfähigen PTEN-Gens umfasst.

6. Proteasom-Inhibitor nach den Patentansprüchen 1 bis 5 zur Verwendung bei der Behandlung von Krebs, wobei die Diagnose des Patienten als empfänglich gegenüber einer Behandlung mit einem Proteasom-Inhibitor ferner das Nachweisen eines Anstiegs des BIM-Spiegels umfasst.

7. Proteasom-Inhibitor zur Verwendung bei der Behandlung von Krebs bei einem Patienten durch Erhöhung der Apoptose in dem Patienten, wobei der Patient ein nicht funktionsfähiges ATAD1-Gen aufweist, wobei der Proteasom-Inhibitor Bortezomib oder Carfilzomib ist.

8. Der Proteasom-Inhibitor nach Patentanspruch 7 zur Verwendung bei der Behandlung von Krebs, wobei der Patient ferner ein nicht funktionsfähiges PTEN-Gen aufweist.

9. Proteasom-Inhibitor nach Patentansprüchen 7 oder 8 zur Verwendung bei der Behandlung von Krebs, wobei der Patient mit einem nicht funktionsfähigen ATAD1-Gen ein Patient ist, bei dem ein Teil oder das gesamte ATAD1-Gen deletiert ist.

10. Der Proteasom-Inhibitor nach den Patentansprüchen 7 bis 9 zur Verwendung bei der Behandlung von Krebs, wobei der Patient mit einem nicht funktionsfähigen PTEN-Gen ein Patient ist, bei dem ein Teil oder das gesamte PTEN-Gen deletiert ist.

11. Der Proteasom-Inhibitor nach den Patentansprüchen 7 bis 10 zur Verwendung bei der Behandlung von Krebs, wobei der Patient Brustkrebs, Lungenkrebs, Darmkrebs, Hirntumor oder Prostatakrebs hat.

## Revendications

1. Inhibiteur du protéasome pour utilisation dans le traitement du cancer chez un sujet, dans lequel le sujet présente un gène ATAD1 non fonctionnel, dans lequel l'inhibiteur du protéasome est le bortézomib ou le carfilzomib.

2. L'inhibiteur du protéasome selon la revendication 1, dans lequel le sujet comprend en outre un gène PTEN non fonctionnel.

3. L'inhibiteur du protéasome selon la revendication 1 ou 2 pour utilisation dans le traitement du cancer, dans lequel une partie ou la totalité du gène ATAD1 est supprimée, et/ou dans lequel une partie ou la totalité du gène PTEN est supprimée.

4. L'inhibiteur du protéasome selon les revendications 1 à 3 pour utilisation dans le traitement du cancer, dans lequel le sujet a déjà été diagnostiqué comme étant sensible au traitement par un inhibiteur du protéasome, qui comprend l'identification du sujet comme ayant un gène ATAD1 non fonctionnel.

5. L'inhibiteur du protéasome selon les revendications 1 à 4, pour utilisation dans le traitement du cancer, dans lequel le diagnostic du sujet comme étant sensible au traitement par un inhibiteur du protéasome comprend en outre l'identification du sujet comme ayant un gène PTEN non fonctionnel.

6. L'inhibiteur du protéasome selon les revendications 1 à 5, pour utilisation dans le traitement du cancer, dans lequel le diagnostic du sujet comme étant sensible au traitement par un inhibiteur du protéasome comprend en outre la détection d'une augmentation des niveaux de BIM.

7. Inhibiteur du protéasome pour utilisation dans le traitement du cancer chez un sujet en augmentant l'apoptose chez ledit sujet, dans lequel le sujet comprend un gène ATAD1 non fonctionnel, dans lequel l'inhibiteur du protéasome est le bortézomib ou le carfilzomib.

8. L'inhibiteur du protéasome selon la revendication 7, pour utilisation dans le traitement du cancer, dans lequel le sujet comprend en outre un gène PTEN non fonctionnel.

9. L'inhibiteur du protéasome selon la revendication 7 ou 8, pour utilisation dans le traitement du cancer, dans lequel le sujet ayant un gène ATAD1 non fonctionnel est un sujet ayant une partie ou la totalité du gène ATAD1 supprimé.

10. L'inhibiteur du protéasome selon les revendications 7 à 9 pour utilisation dans le traitement du cancer, dans lequel le sujet présentant un gène PTEN non fonctionnel est un sujet présentant une partie ou la totalité du gène PTEN supprimé.

11. L'inhibiteur du protéasome selon les revendications 7 à 10 destiné à être utilisé dans le traitement du cancer, dans lequel le sujet atteint d'un cancer présente un cancer du sein, un cancer du poumon, un cancer du côlon, un cancer du cerveau ou un cancer de la prostate.
